# EUROPEAN PATENT APPLICATION

(11) **EP 4 835 060 A1**
(43) Date of publication of application: **04.11.2026**
(21) Application number: 24912994.1
(22) Date of filing: 26.12.2024
(51) Int. Cl.: C12N 7/00, A61K 35/76, A61P 31/04, C12N 7/01, C12N 15/09

(54) **PSEUDOMONAS AERUGINOSA BACTERIOPHAGE**

(30) Priority: 27.12.2023 JP 2023221078
(71) Applicant: Arrowsmith Inc., Tsukuba-shi, Ibaraki 305-0841 (JP); NATIONAL UNIVERSITY CORPORATION TOKAI NATIONAL HIGHER EDUCATION AND RESEARCH SYSTEM, Nagoya-shi, Aichi 464-8601 (JP); Rakuno Gakuen University, Ebetsu-shi, Hokkaido 069-8501 (JP)
(72) Inventor: MATSUKAWA, Koji, Tokyo 103-8411 (JP); ONO, Yutaro, Tokyo 103-8411 (JP); SAKAI, Seishin, Tokyo 103-8411 (JP); NARUSAWA, Megumi, Tokyo 103-8411 (JP); MIYAMOTO, Shuntaro, Tokyo 103-8411 (JP); ANDO, Hiroki, Gifu-shi, Gifu 501-1193 (JP); INABA, Keiko, Gifu-shi, Gifu 501-1193 (JP); MITSUNAKA, Shoichi, Gifu-shi, Gifu 501-1193 (JP); ZAMAN, Most Hushna Ara Naznin, Gifu-shi, Gifu 501-1193 (JP); WASHIZAKI, Ayaka, Gifu-shi, Gifu 501-1193 (JP); IWANO, Hidetomo, Ebetsu-shi, Hokkaido 069-8501 (JP)
(74) Representative: Hoffmann Eitle
(86) International application number: PCT/JP2024/046129
(87) International publication number: WO 2025/143108

(57) **Abstract**

This invention provides a bacteriophage having lytic activity against *Pseudomonas aeruginosa,* wherein a genome of the bacteriophage comprises the nucleic acid sequence of the genome of the bacteriophage identified by Accession No. NITE BP-04040, NITE BP-04031, NITE BP-04033, NITE BP-04036, NITE BP-04034, or NITE BP-04035, or Receipt No. NITE ABP-04200, or NITE ABP-04201. This invention also provides a bacteriophage having lytic activity against *Pseudomonas aeruginosa,* which comprises a nucleic acid sequence encoding a tail fiber gene comprising at least a part of a tail fiber gene derived from another bacteriophage and/or a nucleic acid sequence encoding at least one protein or active domain selected from the group consisting of (a) Pyocin G, (b) PslGh, and (c) PelAh.

## Description

### Technical Field

The present invention relates to a new bacteriophage and a modified bacteriophage having lytic activity against *Pseudomonas aeruginosa,* and uses of these bacteriophages.

### Background Art

*Pseudomonas aeruginosa* is a type of bacterial species of *Pseudomonas* and is a Gram-negative bacterium known to cause hospital-acquired infection. In particular, *Pseudomonas aeruginosa* is a general cause of pneumonia, infection of patients with immunodeficiency, and infection of patients with cystic fibrosis involving respiratory symptoms or the like. Treatment of infection with *Pseudomonas aeruginosa* primarily involves the use of antibiotics. *Pseudomonas aeruginosa* is known as a bacterium capable of acquiring resistance to many antibiotics, and produces a polysaccharide-containing extracellular matrix to form a biofilm. *Pseudomonas aeruginosa* that has formed a biofilm is protected by neutrophil-mediated opsonic actions and antibiotics and, accordingly, it would serve as a problem to be dissolved in treatment (Drugs, 2021; 81 (18): 2117-2131).

A bacteriophage (hereafter, also referred to as a "phage") is a virus whose host is a bacterium. A phage infects a specific bacterium, uses the metabolic mechanism of the host bacterium to propagate its progeny phage, and lyses the host bacterium with the aid of the lytic enzymes encoded by the phage genome. In recent years, phage therapy for infection with *Pseudomonas aeruginosa* using bacteriophages has been attempted (Antibiotics, 2021; 10 (5): 556).

It has been reported that it is possible to change host ranges by preparing a bacteriophage resulting from substitution of a part of the tail fiber gene of *Pseudomonas aeruginosa-*infecting bacteriophage with the tail fiber gene of another bacteriophage (WO 2016/055585 A).

A bacteriophage can be genetically recombinated by a synthetic biological means. For example, it is possible to delete or insert a particular gene. The same applies to *Pseudomonas aeruginosa-*infecting bacteriophages (Proceedings of the National Academy of Sciences of the United States of America, 2022; 119 (48): e2206739119).

As proteins that inhibit *Pseudomonas aeruginosa* biofilm formation and destruct the *Pseudomonas aeruginosa* biofilm, *Pseudomonas aeruginosa-*derived glycoside hydrolases; i.e., PelA and PslG, and their respective active domains PelAh and PslGh, are known. Such hydrolases degrade extracellular polysaccharides Pel and Psl, respectively, produced by *Pseudomonas aeruginosa* (Science Advances, 2016; 2 (5): e1501632). Dependency of biofilms formed by *Pseudomonas aeruginosa* strains on Pel and Psl varies depending on bacterial strains, and a strain that forms a Pel-dominant matrix, a strain that forms a Psl-dominant matrix, a Pel- and Psl-redundant strain, and the like exist (Environmental microbiology, 2012; 14 (8): 1913-1928). Accordingly, the capacity of PelA, PelAh, PslG, and PslGh to degrade *Pseudomonas aeruginosa* biofilms is deduced to vary among *Pseudomonas aeruginosa* strains.

Bacteriocin is a protein produced by bacteria that generally kills by the same bacteria species. As a factor exhibiting antibacterial activity against *Pseudomonas aeruginosa,* bacteriocin derived from *Pseudomonas aeruginosa,* Pyocin G (hereafter, also referred to as "PyoG"), is known (Journal of Molecular Biology, 2020; 432 (13): 3869-3880).

It has been reported that a particular gene may be inserted into a bacteriophage genome to change activity of the bacteriophage as described below. That is, a genetically engineered bacteriophage resulting from artificial insertion of a gene encoding an extracellular polysaccharide-degrading enzyme into the genome of the *E*. *coli*-infecting bacteriophage T7 is capable of removing a biofilm formed by *E. coli* (Proceedings of the National Academy of Sciences of the United States of America, 2007; 104 (27): 11197-11202). Also, an alginate lyase may be inserted into the genome of a *Pseudomonas aeruginosa-*infecting bacteriophage to degrade alginic acid as a biofilm constituent (WO 2023/015195 A). In addition, bacteriocin may be inserted into the genome of a *E. coli-, Klebsiella-,* or *Enterococcus-*infecting bacteriophage to allow the bacteriophage to exert antibacterial activity against other relevant bacterial species (WO 2023/052628 A, Nat. Commun., 2023; 14: 4337). On the other hand, there has been no report concerning insertion of bacteriocin into the genome of a *Pseudomonas aeruginosa-*infecting bacteriophage.

In general, a cocktail of two or more different types of bacteriophages may be used in bacteriophage therapy for the purpose of host range expansion or prevention from development of bacteriophage resistance. In addition, it has been demonstrated that antibacterial activity of *Pseudomonas aeruginosa-*infecting bacteriophage would be improved by, for example, mixing the bacteriophage with a bacteriophage of a different genetic lineage (Scientific Reports, 2023; 13 (1): 8921).

In bacteria including *Pseudomonas aeruginosa,* lysogenic bacteriophages may be lysogenized and inserted into the host genome (the lysogenized bacteriophages also referred to as "prophages"). In the *Pseudomonas aeruginosa* PAO1 strain, for example, two filamentous phages referred to as Pf4 and Pf6 are lysogenized. A method for obtaining a strain resulting from deletion of such phages from the host strain has been reported (Journal of Bacteriology, 2024; 206 (5): e0040223).

A prophage that is lysogenized in bacteria may encode a gene that protects the host bacteria to protect the host bacteria from infection with a competitive bacteriophage. A case in which proliferation of a competitive bacteriophage is thus inhibited has been reported (Nat. Commum., 2024; 15 (1): 1644).

### Citation List

### Patent Literature

Patent Literature 1: WO 2016/055585 A
Patent Literature 2: WO 2023/015195 A
Patent Literature 3: WO 2023/052628 A

### Non Patent Literature

Non Patent Literature 1: Science Advances, 2016; 2 (5): e1501632
Non Patent Literature 2: Journal of Molecular Biology, 2020; 432 (13): 3869-3880
Non Patent Literature 3: Proceedings of the National Academy of Sciences of the United States of America, 2007; 104 (27): 11197-11202
Non Patent Literature 4: Nat. Commun., 2023; 14: 4337
Non Patent Literature 5: Scientific Reports, 2023; 13 (1): 8921
Non Patent Literature 6: Journal of Bacteriology, 2024; 206 (5): e0040223
Non Patent Literature 7: Nat. Commun., 2024; 15 (1): 1644

### Summary of Invention

### Technical Problem

An object of the present invention is to provide a bacteriophage having lytic activity against bacteria of *Pseudomonas* including *Pseudomonas aeruginosa.* Another object of the present invention is to provide a means and a method, particularly a bacteriophage and a pharmaceutical composition, for preventing or treating infection with bacteria of *Pseudomonas*, such as infection with *Pseudomonas aeruginosa.*

### Solution to Problem

As a result of creative studies in producing a bacteriophage, the present inventors obtained new bacteriophages and found that the bacteriophages have lytic activity against *Pseudomonas aeruginosa* (Examples 1, 8, 9, and 10). In addition, we produced bacteriophages, wherein a tail fiber gene of the bacteriophages has been replaced and found that such bacteriophages would have lytic activity against a more extensive range of *Pseudomonas aeruginosa* (Example 2). We produced bacteriophages comprising a glycoside hydrolase introduced thereinto (Example 3), we also produced bacteriophages comprising bacteriocin introduced thereinto (Example 4), and we verified that such bacteriophages had lytic activity against *Pseudomonas aeruginosa* (Examples 3 and 4). We found that new bacteriophages, modified bacteriophages, and a cocktail of two or more different types of bacteriophages had biofilm-degrading activity (Examples 5, 6, and 12) and found that the new bacteriophages, the modified bacteriophages, and the cocktail had lytic activity against *Pseudomonas aeruginosa* (Examples 7 to 9, 11, and 13). We produced host bacteria lacking the prophage-derived gene and found that such host bacteria would efficiently amplify bacteriophages (Example 14). Based on these findings, the present invention has been accomplished.

Specifically, the present invention may encompass the following inventions as materials or methods that are medically or industrially useful.
[1] A bacteriophage selected from (1) to (8) below:
   (1) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04040;
   (2) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04031;
   (3) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04033;
   (4) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04036;
   (5) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04034;
   (6) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04035;
   (7) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04200; and
   (8) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04201.
[2] The bacteriophage according to [1] selected from (1) to (8) below:
   (1) a bacteriophage having lytic activity against *Pseudomonas aeruginosa,* wherein a genome of the bacteriophage comprises the nucleic acid sequence of the genome of the bacteriophage identified by Accession No. NITE BP-04040;
   (2) a bacteriophage having lytic activity against *Pseudomonas aeruginosa,* wherein a genome of the bacteriophage comprises the nucleic acid sequence of the genome of the bacteriophage identified by Accession No. NITE BP-04031;
   (3) a bacteriophage having lytic activity against *Pseudomonas aeruginosa,* wherein a genome of the bacteriophage comprises the nucleic acid sequence of the genome of the bacteriophage identified by Accession No. NITE BP-04033;
   (4) a bacteriophage having lytic activity against *Pseudomonas aeruginosa,* wherein a genome of the bacteriophage comprises the nucleic acid sequence of the genome of the bacteriophage identified by Accession No. NITE BP-04036;
   (5) a bacteriophage having lytic activity against *Pseudomonas aeruginosa,* wherein a genome of the bacteriophage comprises the nucleic acid sequence of the genome of the bacteriophage identified by Accession No. NITE BP-04034;
   (6) a bacteriophage having lytic activity against *Pseudomonas aeruginosa,* wherein a genome of the bacteriophage comprises the nucleic acid sequence of the genome of the bacteriophage identified by Accession No. NITE BP-04035;
   (7) a bacteriophage having lytic activity against *Pseudomonas aeruginosa,* wherein a genome of the bacteriophage comprises the nucleic acid sequence of the genome of the bacteriophage identified by Receipt No. NITE ABP-04200; and
   (8) a bacteriophage having lytic activity against *Pseudomonas aeruginosa,* wherein a genome of the bacteriophage comprises the nucleic acid sequence of the genome of the bacteriophage identified by Receipt No. NITE ABP-04201.
[3] The bacteriophage according to [1] or [2] selected from (1) to (8) below:
   (1) a bacteriophage, wherein a genome of the bacteriophage consists of the nucleic acid sequence of the genome of the bacteriophage identified by Accession No. NITE BP-04040;
   (2) a bacteriophage, wherein a genome of the bacteriophage consists of the nucleic acid sequence of the genome of the bacteriophage identified by Accession No. NITE BP-04031;
   (3) a bacteriophage, wherein a genome of the bacteriophage consists of the nucleic acid sequence of the genome of the bacteriophage identified by Accession No. NITE BP-04033;
   (4) a bacteriophage, wherein a genome of the bacteriophage consists of the nucleic acid sequence of the genome of the bacteriophage identified by Accession No. NITE BP-04036;
   (5) a bacteriophage, wherein a genome of the bacteriophage consists of the nucleic acid sequence of the genome of the bacteriophage identified by Accession No. NITE BP-04034;
   (6) a bacteriophage, wherein a genome of the bacteriophage consists of the nucleic acid sequence of the genome of the bacteriophage identified by Accession No. NITE BP-04035;
   (7) a bacteriophage, wherein a genome of the bacteriophage consists of the nucleic acid sequence of the genome of the bacteriophage identified by Receipt No. NITE ABP-04200; and
   (8) a bacteriophage, wherein a genome of the bacteriophage consists of the nucleic acid sequence of the genome of the bacteriophage identified by Receipt No. NITE ABP-04201.
[4] A bacteriophage selected from (1) to (8) below:
   (1) the bacteriophage identified by Accession No. NITE BP-04040 or a passage strain thereof;
   (2) the bacteriophage identified by Accession No. NITE BP-04031 or a passage strain thereof;
   (3) the bacteriophage identified by Accession No. NITE BP-04033 or a passage strain thereof;
   (4) the bacteriophage identified by Accession No. NITE BP-04036 or a passage strain thereof;
   (5) the bacteriophage identified by Accession No. NITE BP-04034 or a passage strain thereof;
   (6) the bacteriophage identified by Accession No. NITE BP-04035 or a passage strain thereof;
   (7) the bacteriophage identified by Receipt No. NITE ABP-04200 or a passage strain thereof; and
   (8) the bacteriophage identified by Receipt No. NITE ABP-04201 or a passage strain thereof.
[5] A bacteriophage having lytic activity against *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa.*
[6] The bacteriophage having lytic activity against *Pseudomonas aeruginosa* according to [5], wherein a genome of the bacteriophage (a) comprises the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa.*
[7] The bacteriophage having lytic activity against *Pseudomonas aeruginosa* according to [5], wherein a genome of the bacteriophage (a) consists of the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa.*
[8] The bacteriophage according to [5], wherein a tail fiber gene derived from another bacteriophage comprises the nucleic acid sequence shown in SEQ ID NO: 6 or a nucleic acid sequence having 90% or more identity to SEQ ID NO: 6.
[9] A bacteriophage having lytic activity against *Pseudomonas aeruginosa,* wherein a genome of the bacteriophage comprises a nucleic acid sequence(s) encoding at least one protein or active domain selected from (a) to (c) below:
   (a) Pyocin G;
   (b) PslGh; and
   (c) PelAh.
[10] The bacteriophage according to [9], wherein a genome of the bacteriophage comprises a nucleic acid sequence(s) encoding at least two proteins or active domains selected from (a) to (c) below:
   (a) Pyocin G;
   (b) PslGh; and
   (c) PelAh.
[11] The bacteriophage according to [9], wherein a genome of the bacteriophage comprises a nucleic acid sequence(s) encoding proteins or active domains (a) to (c) below:
   (a) Pyocin G;
   (b) PslGh; and
   (c) PelAh.
[12] A pharmaceutical composition comprising the bacteriophage according to any one of [1] to [11] and a pharmaceutically acceptable excipient.
[13] A pharmaceutical composition comprising at least two types of the bacteriophages according to any one of [1] to [11] and a pharmaceutically acceptable excipient.
[14] A pharmaceutical composition comprising three types of the bacteriophages according to any one of [1] to [11] and a pharmaceutically acceptable excipient.
[15] A pharmaceutical composition comprising four types of the bacteriophages according to any one of [1] to [11] and a pharmaceutically acceptable excipient.
[16] A pharmaceutical composition comprising five types of the bacteriophages according to any one of [1] to [11] and a pharmaceutically acceptable excipient.
[17] The pharmaceutical composition according to any one of [12] to [16], which is a pharmaceutical composition for preventing or treating infection with *Pseudomonas aeruginosa.*
[18] The pharmaceutical composition according to [17], wherein the infection with *Pseudomonas aeruginosa* is respiratory infection.
[19] A bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04032 except for the region of the tail fiber gene and comprises nucleic acid sequences (A) and (B) below:
   (A) a nucleic acid sequence encoding the tail fiber gene derived from bacteriophage JG024; and
   (B) a nucleic acid sequence encoding PslGh.
[20] A bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6, or a nucleic acid sequence having 90% or more identity to SEQ ID NO: 6; and
   (B) the nucleic acid sequence shown in SEQ ID NO: 4, or a nucleic acid sequence having 90% or more identity to SEQ ID NO: 4 and encoding a protein having Psl-degrading activity.
[21] A bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 10 or SEQ ID NO: 11.
[22] A pharmaceutical composition comprising a bacteriophage and a pharmaceutically acceptable excipient, wherein the bacteriophage comprises the bacteriophages (i) to (iv) below:
   (i) the bacteriophage identified by Accession No. NITE BP-04037 or Receipt No. NITE ABP-04201 or a passage strain thereof;
   (ii) the bacteriophage identified by Accession No. NITE BP-04031 or a passage strain thereof;
   (iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
      (A) the nucleic acid sequence shown in SEQ ID NO: 6; and
      (B) the nucleic acid sequence shown in SEQ ID NO: 10 or SEQ ID NO: 11; and
   (iv) the bacteriophage identified by Accession No. NITE BP-04040 or Receipt No. NITE ABP-04200 or a passage strain thereof.
[23] The pharmaceutical composition according to [22], which is a pharmaceutical composition for preventing or treating infection with *Pseudomonas aeruginosa.*
[24] The pharmaceutical composition according to [23], wherein the infection with *Pseudomonas aeruginosa* is respiratory infection.
[25] A method for producing a bacteriophage having lytic activity against *Pseudomonas aeruginosa,* the method comprising a step of culturing the bacteriophage in a host bacterium from which a prophage-derived gene has been deleted.

### Advantageous Effects of Invention

The bacteriophage, the modified bacteriophage, and the pharmaceutical composition of the present invention exhibit lytic activity against *Pseudomonas aeruginosa* and the bacteriophage, the modified bacteriophage, and the pharmaceutical composition of the present invention are expected to be useful for preventing or treating infection with *Pseudomonas aeruginosa* including lung infection.

### Brief Description of Drawings

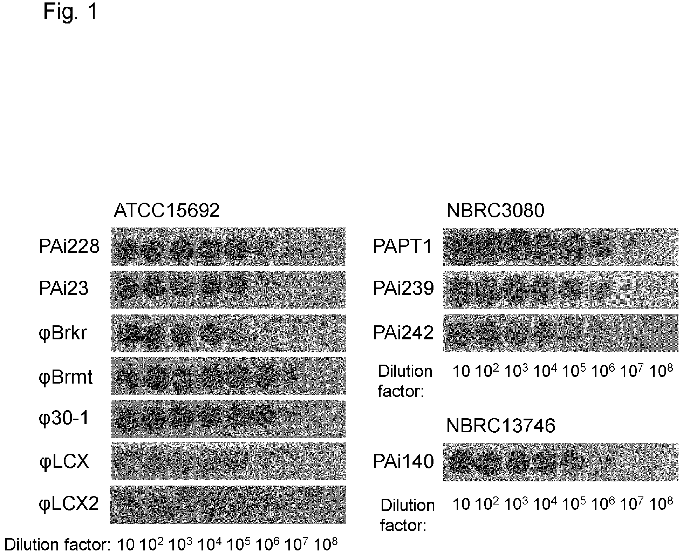

[Fig. 1] Fig. 1 shows photographs demonstrating lytic activity of naturally-occurring bacteriophages. Fig. 1 shows images of lytic plaques appeared after the *Pseudomonas aeruginosa* ATCC15692 strain, NBRC3080 strain, and NBRC13746 strain were overlaid on an agar plate as host strains and the bacteriophage dilution series was added dropwise to the plate.

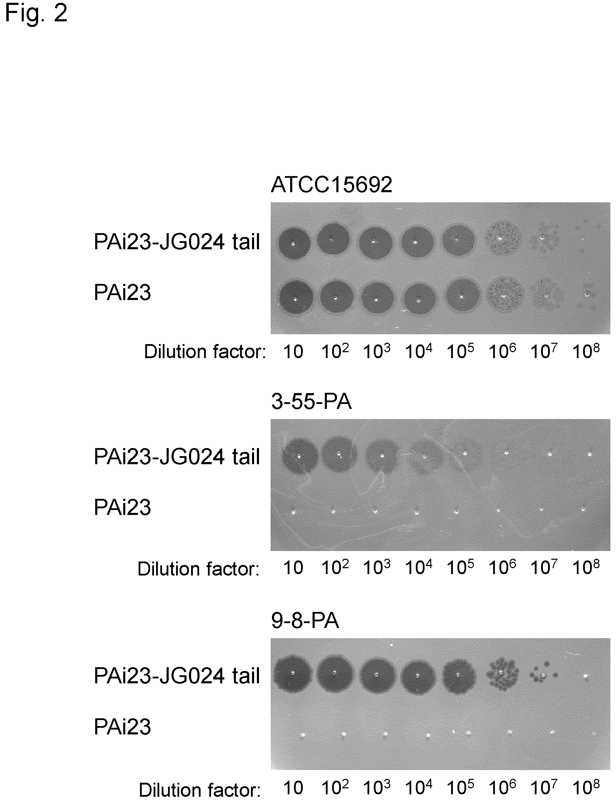

[Fig. 2] Fig. 2 shows photographs demonstrating the lytic activity of the phages PAi23-JG024 tail and PAi23. Fig. 2 shows images of lytic plaques appeared after the *Pseudomonas aeruginosa* ATCC15692 strain, the clinical isolate 3-55-PA strain, and the clinical isolate 9-8-PA strain were overlaid on an agar plate as host strains and the bacteriophage dilution series was added dropwise to the plate.

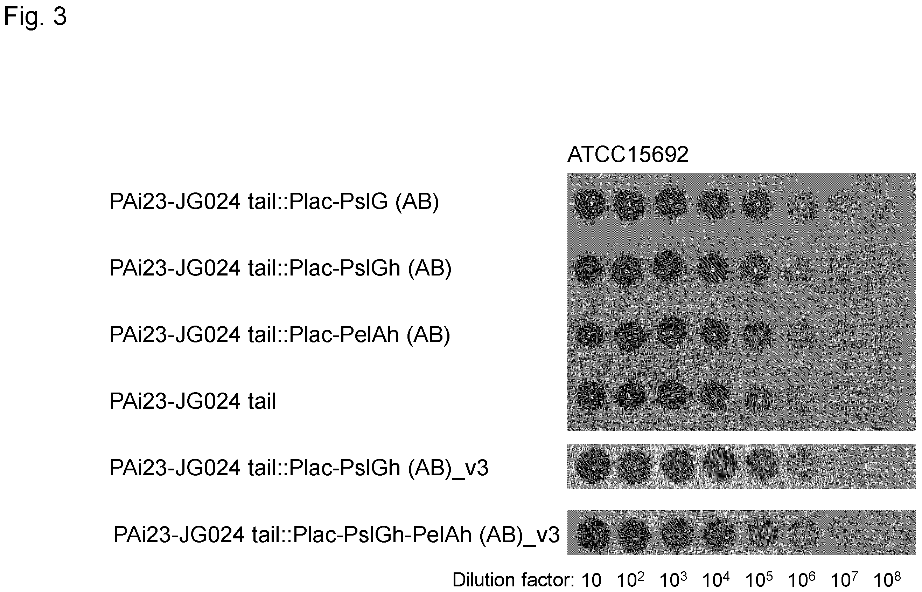

[Fig. 3] Fig. 3 shows photographs demonstrating lytic activity of phages PAi23-JG024 tail::Plac-PelAh (AB), PAi23-JG024 tail::Plac-PslGh (AB), PAi23-JG024 tail::Plac-PslG(AB), PAi23-JG024 tail::Plac-PslGh (AB)_v3, and PAi23-JG024 tail::Plac-PslGh-PelAh (AB)_v3. Fig. 3 shows images of lytic plaques appeared after the *Pseudomonas aeruginosa* ATCC15692 strain was overlaid on an agar plate as the host strain and the bacteriophage dilution series was added dropwise to the plate.

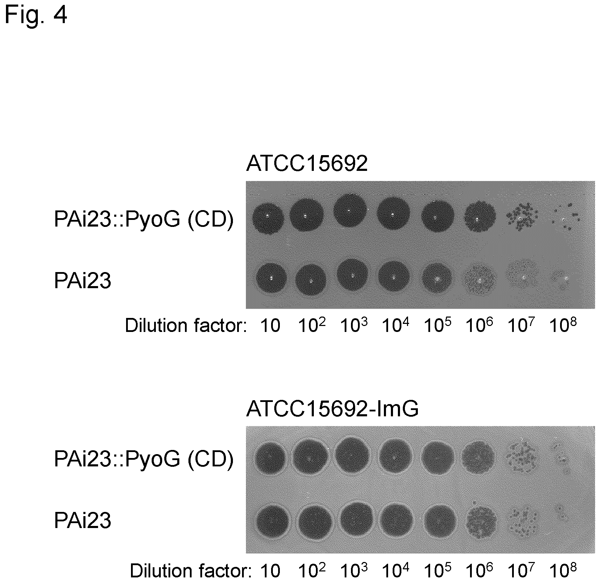

[Fig. 4] Fig. 4 shows photographs demonstrating lytic activity of the PAi23::PyoG (CD) phage. Fig. 4 shows images of lytic plaques appeared after the *Pseudomonas aeruginosa* ATCC15692 strain and the ATCC15692 strain expressing the ImG gene (ATCC15692-ImG) were overlaid on an agar plate as host strains and the bacteriophage dilution series was added dropwise to the plate.

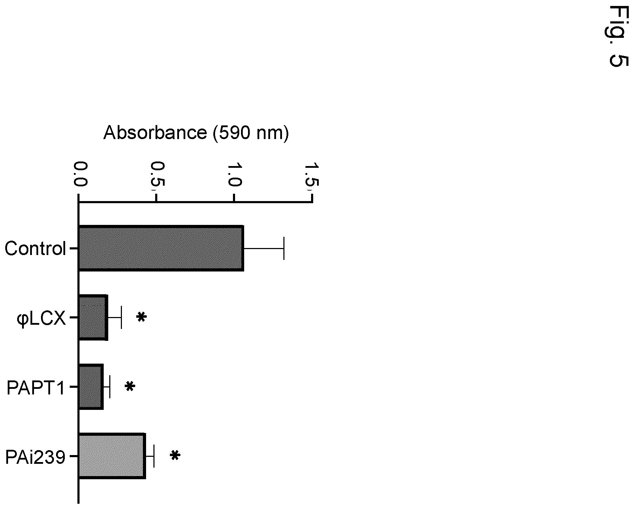

[Fig. 5] Fig. 5 shows a graph demonstrating that φLCX, PAPT1, and PAi239 each have activity of reducing the amount of biofilms. The *Pseudomonas aeruginosa* ATCC15692 strain was used to form biofilms for 24 hours, media each containing a relevant bacteriophage was brought into contact with the biofilms, and the amount of the biofilms was quantified via crystal violet staining 6 hours later. An error bar indicates a standard error.

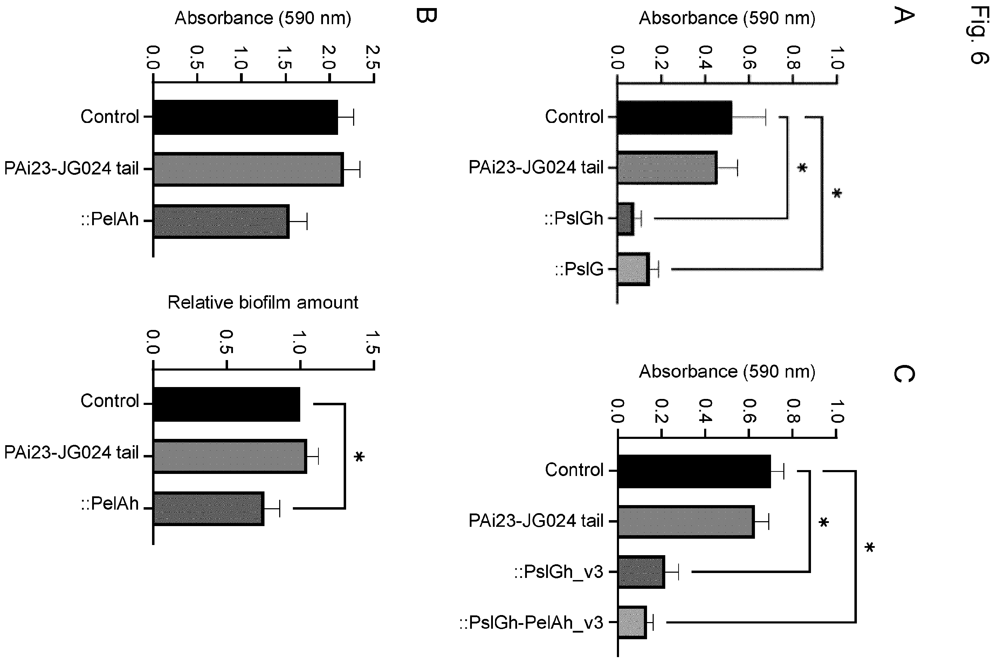

[Fig. 6] Fig. 6A shows a graph demonstrating that PAi23-JG024 tail::Plac-PslGh (AB) and PAi23-JG024 tail::Plac-PslG (AB) phages each have activity of reducing the amount of biofilms. The biofilms formed as a result of culture of the *Pseudomonas aeruginosa* ATCC15692 strain for 24 hours were each brought into contact with a bacteriophage-containing medium for 6 hours, and the amount of the biofilms in the medium was quantified by crystal violet staining. An error bar indicates a standard error. Fig. 6B shows graphs demonstrating that PAi23-JG024 tail::Plac-PelAh (AB) phage has activity of reducing the amount of biofilms. The biofilms formed as a result of culture of the respiratory clinical isolate 28-42-PA strain for 24 hours were each brought into contact with a bacteriophage-containing medium for 6 hours, and the amount of the biofilms in the medium was quantified by crystal violet staining (the left graph). A graph showing the amount of biofilms converted to relative values is also shown (the right graph). An error bar indicates a standard error. Fig. 6C shows a graph demonstrating that PAi23-JG024 tail::Plac-PslGh (AB)_v3 and PAi23-JG024 tail::Plac-PslGh-PelAh (AB)_v3 phages each have activity of reducing the amount of biofilms. The biofilms formed as a result of culture of the *Pseudomonas aeruginosa* ATCC15692 strain for 24 hours were each brought into contact with a bacteriophage-containing medium for 6 hours, and the amount of the biofilms in the medium was quantified by crystal violet staining. An error bar indicates a standard error.

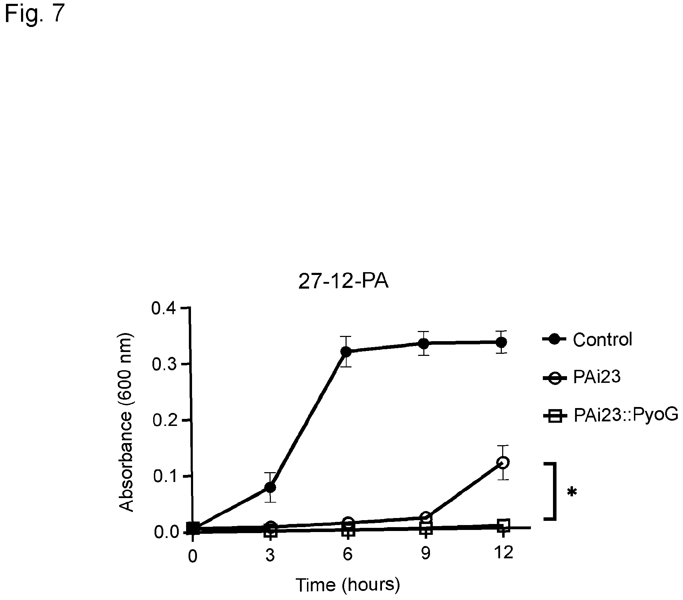

[Fig. 7] Fig. 7 shows a graph demonstrating the growth curves of *Pseudomonas aeruginosa* in liquid culture. The growth curves demonstrates that effects of inhibiting the growth of the respiratory clinical isolate 27-12-PA strain are observed with the addition of the PAi23 phage and that effects of inhibiting the growth of the respiratory clinical isolate 27-12-PA strain are maintained for a longer period of time with the addition of the PAi23::PyoG (CD) phage compared with the addition of the PAi23 phage. An error bar indicates a standard error.

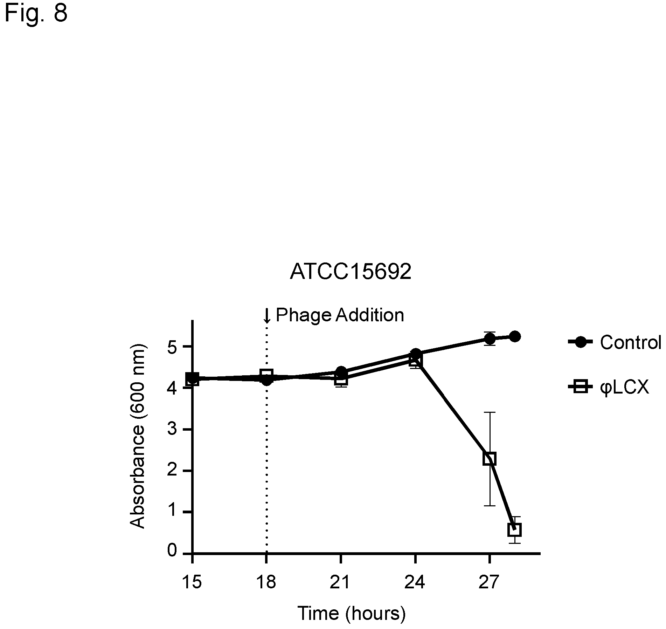

[Fig. 8] Fig. 8 shows a graph demonstrating antibacterial activity of the φLCX phage against *Pseudomonas aeruginosa* after overnight culture. The *Pseudomonas aeruginosa* ATCC15692 strain was cultured in a glass test tube and φLCX was added 18 hours later. The graph demonstrates a change in the absorbance at 600 nm. An error bar indicates a standard error.

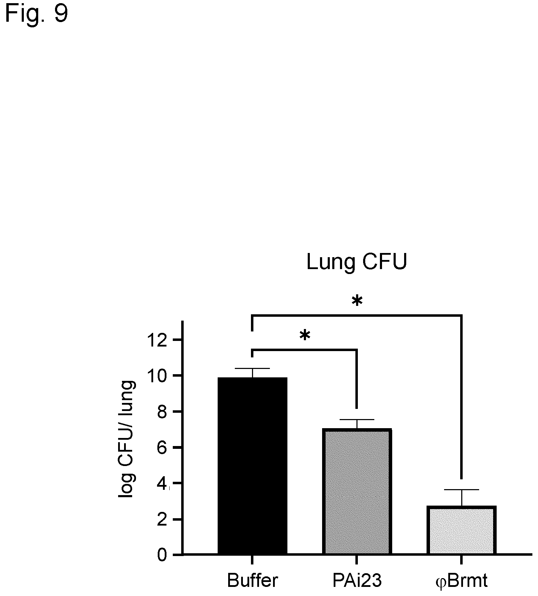

[Fig. 9] Fig. 9 shows a graph demonstrating antibacterial activity of a single bacteriophage in a mouse model of *Pseudomonas aeruginosa* infection. The mouse was infected with the *Pseudomonas aeruginosa* ATCC15692 strain through the nose and then with PAi23 or φBrmt was administered intranasally 30 minutes thereafter. The graph demonstrates the viable bacterial count in the lung 24 hours after infection (log CFU/lung) (CFU: colony forming unit). An error bar indicates a standard error.

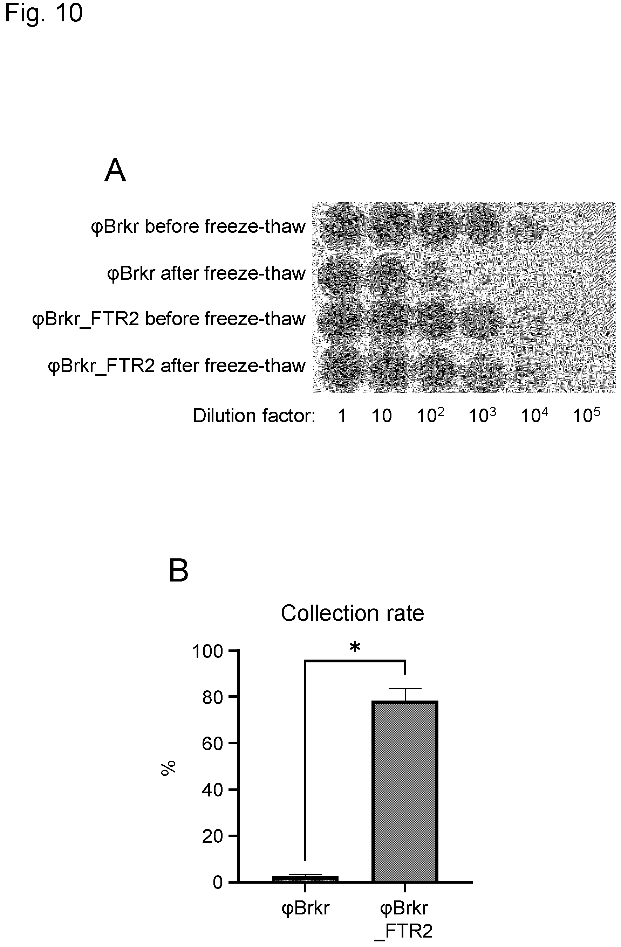

[Fig. 10] Fig. 10A shows a photograph demonstrating the results of quantification of the plaque forming unit per mL (PFU/mL) of the φBrkr phage and that of the freeze-thaw resistant strain of the φBrkr phage (φBrkr_FTR2) before and after freeze-thaw treatment. Fig. 10A shows an image of lytic plaques appeared after the *Pseudomonas aeruginosa* ATCC15692ΔPf4/Pf6 strain was overlaid on an agar plate as the host strain and the bacteriophage dilution series was added dropwise to the plate. Fig. 10B shows a graph demonstrating that the activity of φBrkr was reduced to a significant extent after the freeze-thaw treatment while φBrkr_FTR2 had acquired freeze-thaw resistance. An error bar indicates a standard error.

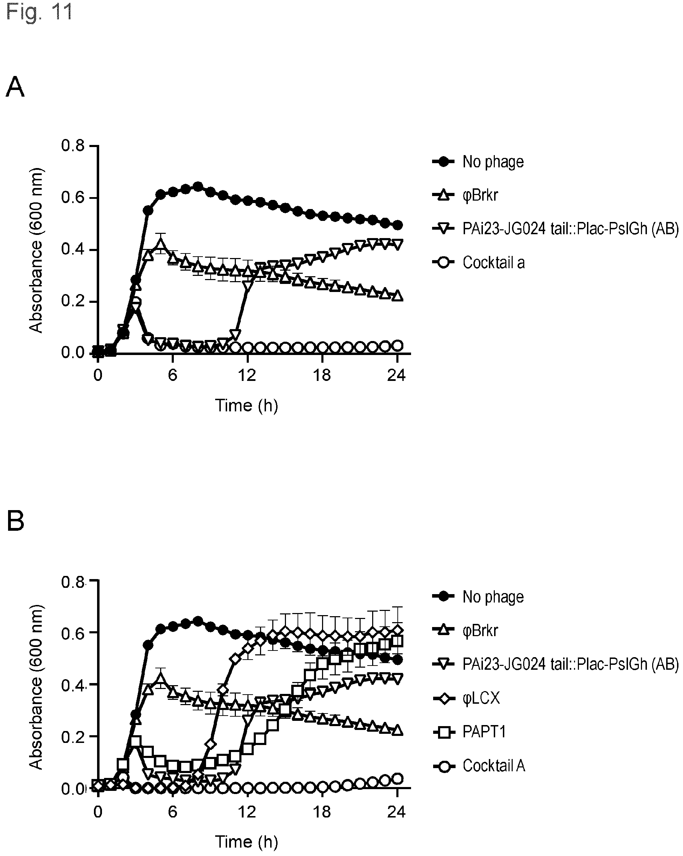

[Fig. 11] Fig. 11 shows graphs demonstrating that effects of inhibiting the growth of *Pseudomonas aeruginosa* would be extended by preparing a bacteriophage cocktail. The graphs show representative examples of the growth curves obtained after a single bacteriophage or a cocktail of two or four types of bacteriophages was prepared to adjust MOI to 0.0001 and allowed to infect the *Pseudomonas aeruginosa* ATCC15692 strain. An error bar indicates a standard error.

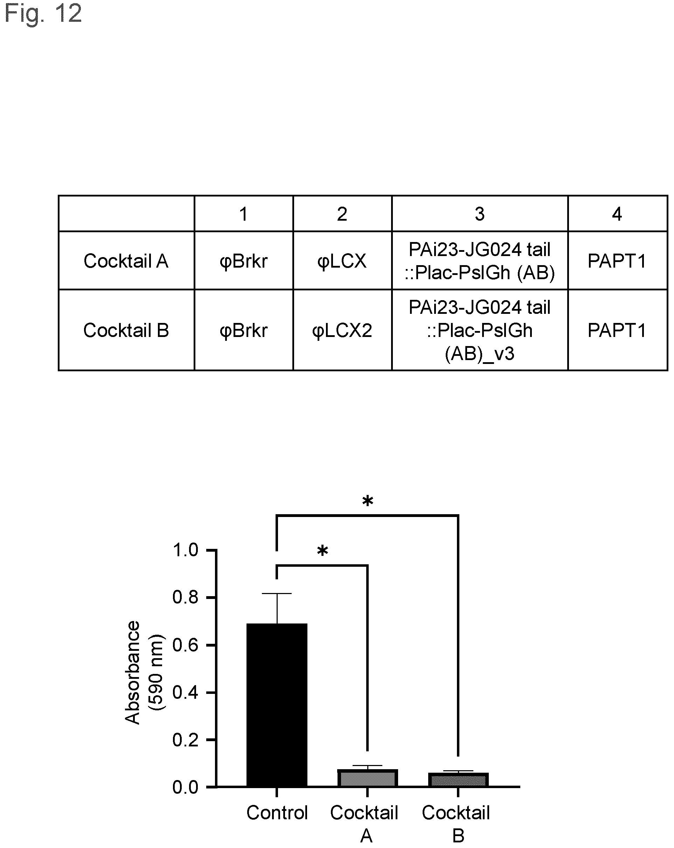

[Fig. 12] Fig. 12 shows a graph demonstrating that the bacteriophage cocktails have activity of reducing the amount of biofilms. The biofilms formed as a result of culture of the *Pseudomonas aeruginosa* ATCC15692 strain for 24 hours were each brought into contact with a medium containing the bacteriophage cocktail (Cocktail A or B) for 6 hours, and the amount of the biofilms in the medium was quantified by crystal violet staining. An error bar indicates a standard error.

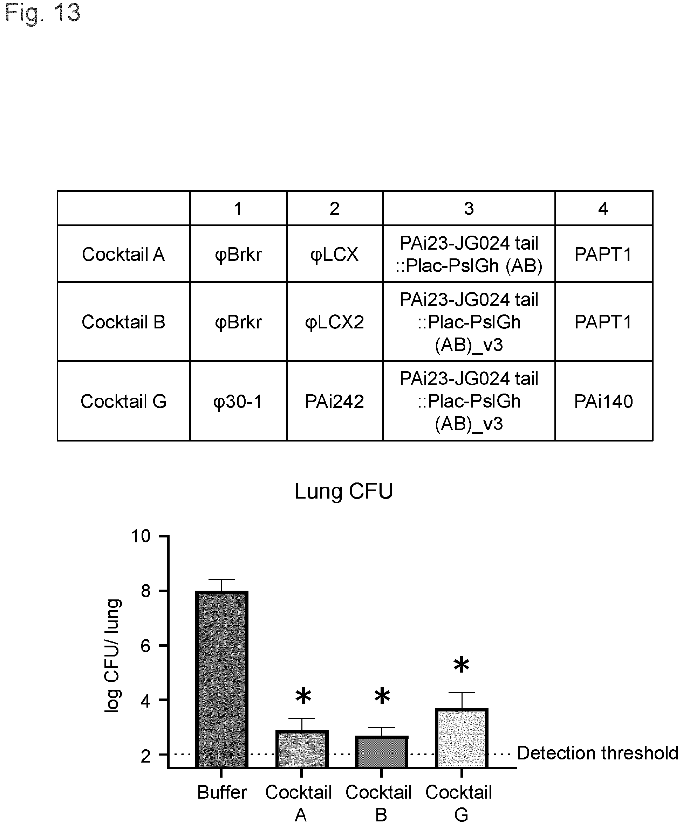

[Fig. 13] Fig. 13 shows a graph demonstrating antibacterial activity of bacteriophage cocktails in a mouse model of *Pseudomonas aeruginosa* infection. The mouse was infected with the respiratory clinical isolate 27-12-PA strain by oropharyngeal aspiration and the phage cocktail (Cocktail A, B, or G) was administered 1 hour later. The graph demonstrates the viable bacterial count in the lung approximately 22.5 hours after infection (log CFU/lung). An error bar indicates a standard error.

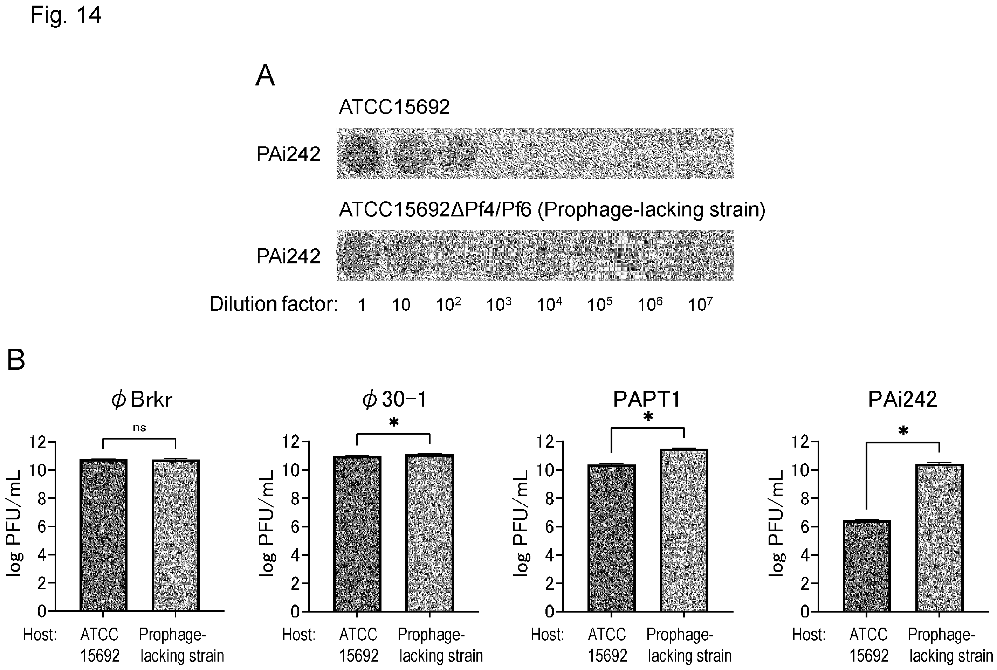

[Fig. 14] Fig. 14A shows images of lytic plaques appeared after the *Pseudomonas aeruginosa* ATCC15692 strain and the ATCC15692ΔPf4/Pf6 strain, which is a prophage-lacking strain of the *Pseudomonas aeruginosa* ATCC15692 strain, were overlaid on an agar plate as host strains and the PAi242 phage dilution series was added dropwise to the plate. Fig. 14B shows graphs demonstrating the titer values of the bacteriophage solutions obtained after the bacteriophages had been cultured using the *Pseudomonas aeruginosa* ATCC15692 strain or the prophage-lacking strain thereof for 6 hours. An error bar indicates a standard error.

### Description of Embodiments

This application claims the priority of Japanese Patent Application No. 2023-221078, filed on December 27, 2023, the entire contents of which are incorporated herein by reference.

Hereinafter, the present invention will be described in detail. The following embodiments are examples for explaining the present invention and are not intended to limit the present invention only to these embodiments. The present invention can be embodied in various embodiments without departing from the gist thereof.

### < 1. Bacteriophage of the present invention >

In one aspect, the present invention provides a new bacteriophage (hereafter also referred to as "the bacteriophage of the present invention). The bacteriophage of the present invention is a bacteriophage having lytic activity against bacteria of *Pseudomonas,* particularly *Pseudomonas aeruginosa.*

The φLCX strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04040).

The PAPT1 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04031).

The PAi140 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04033).

The PAi242 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04036).

The PAi228 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04034).

The PAi239 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04035).

The φLCX2 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and received on November 13, 2024 (Receipt No. NITE ABP-04200).

The φBrkr_FTR2 strain, which is the bacteriophage of the present invention, was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and received on November 13, 2024 (Receipt No. NITE ABP-04201).

In one embodiment, the bacteriophage of the present invention may include the following bacteriophages:
(1) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04040;
(2) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04031;
(3) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04033;
(4) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04036;
(5) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04034;
(6) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04035;
(7) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04200; and
(8) a bacteriophage having lytic activity against bacteria of *Pseudomonas,* such as *Pseudomonas aeruginosa*, wherein a genome of the bacteriophage comprises a nucleic acid sequence having 90% or more identity to the nucleic acid sequence of the genome of the bacteriophage identified by Receipt No. NITE ABP-04201.

The term "bacteriophage" or "phage" used herein refers to a phage particle containing a genome packaged in an envelope or capsid, and the term encompasses the entire phage particle, a part of a phage having equivalent functions (e.g., a phage head), and an aggregate of phage constituents. In particular, an isolated phage refers to a phage that is separated from the natural environment, and is grown, purified, or cultured.

The "identity" used herein means the identity of the nucleotide sequence or amino acid sequence determined by the technique known in the art. As the method of sequence alignment, publicly available alignment software can be used. Examples include, but are not limited to, CLUSTAL W program (Nucleic Acids Research, 1994; 22 (22): 4673-80), FASTA program (Proceedings of the National Academy of Sciences of the United States of America, 1988; 85 (8): 2444-8), and BLAST programs (e.g., BLASTP, BLASTN, BLASTX, TBLASTN, and TBLASTX) (Journal of Molecular Biology, 1990; 215 (3): 403-10). The "identity" means the value Identity obtained using parameters prepared by default by, for example, NEEDLE program (Journal of Molecular Biology, 1970, 48 (3), 443-53) search as a specific method of sequence alignment. The parameters are as follows.
Gap penalty = 10
Extend penalty = 0.5
Matrix = EBLOSUM62

The term "bacteria of *Pseudomonas*" (also referred to as "*Pseudomonas* bacteria" herein) refers to bacteria classified as Gram-negative aerobic rod-shaped bacteria that are known in the art. In one embodiment, *Pseudomonas* bacteria may be bacteria that would cause or be responsible for infection, and examples thereof include *Pseudomonas aeruginosa* (*P. aeruginosa), Pseudomonas paucimobilis (P. paucimobilis), Pseudomonas putida (P. putida), Pseudomonas fluorescens* (*P. fluorescens*)*,* and *Pseudomonas acidovorans* (*P. acidovorans*). Bacteria of *Pseudomonas* are known to protect themselves from external or physical stress by forming biofilms by secreting an extracellular polysaccharide, such as alginic acid.

As used herein, "having lytic activity against bacteria of *Pseudomonas*" or "having lytic activity against *Pseudomonas aeruginosa*" means to show lytic plaques against at least one strain of bacteria of *Pseudomonas* or at least one strain of *Pseudomonas aeruginosa.* Confirmation of lytic activity against these bacteria can be performed using a method and means known in the art. For example, whether or not a bacteriophage has lytic activity can be confirmed using the method described in Example 1 herein by confirming whether or not about 10¹⁰ PFU (plaque forming unit)/mL to 10⁵ PFU/mL of bacteriophage (or a mixture of a plurality of bacteriophages) exhibits lytic plaques against *Pseudomonas aeruginosa* or bacteria of *Pseudomonas.*

In one embodiment, the bacteriophage of the present invention may be resistant to freeze-thaw. For example, the bacteriophage of the present invention may include the following bacteriophage:
a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04201; and
the bacteriophage is resistant to freeze-thaw.

Herein, the phrase "resistant to freeze-thaw" means that the bacteriophage is maintained at a desired level even after a freeze-thaw treatment although it is known that a part of the bacteriophage may be killed or damaged by a freeze-thaw treatment. For example, the number of bacteriophages after a freeze-thaw treatment is maintained at a level substantially equivalent to that before the freeze-thaw treatment, or is maintained at least 10%, at least 20%, at least 30%, at least 40%, at least 50%, at least 60%, at least 70%, at least 80%, or at least 90% or more compared to that before the freeze-thaw treatment. Whether or not a bacteriophage is resistant to freeze-thaw can be confirmed by methods and means known in the art. For example, whether a bacteriophage is resistant to freeze-thaw can be confirmed using the method described in Example 10 herein to check whether or not the bacteriophage shows a reduction in count after the freeze-thaw treatment. Alternatively, whether or not a bacteriophage is resistant to freeze-thaw can also be confirmed by checking whether or not the activity of the bacteriophage (for example, the lytic activity against *Pseudomonas aeruginosa*) is maintained at a desired level after the freeze-thaw treatment. For example, when the bacteriophage maintains the activity at a level substantially equivalent to that before the freeze-thaw treatment, or maintains the activity at least 10%, at least 20%, at least 30%, at least 40%, at least 50%, at least 60%, at least 70%, at least 80%, or at least 90% or more compared to that before the freeze-thaw treatment, the bacteriophage is considered to be resistant to freeze-thaw.

In one embodiment, the bacteriophage of the present invention may include the following bacteriophages:
(1) a bacteriophage having lytic activity against *Pseudomonas aeruginosa,* wherein a genome of the bacteriophage comprises the nucleic acid sequence of the genome of the bacteriophage identified by Accession No. NITE BP-04040;
(2) a bacteriophage having lytic activity against *Pseudomonas aeruginosa,* wherein a genome of the bacteriophage comprises the nucleic acid sequence of the genome of the bacteriophage identified by Accession No. NITE BP-04031;
(3) a bacteriophage having lytic activity against *Pseudomonas aeruginosa,* wherein a genome of the bacteriophage comprises the nucleic acid sequence of the genome of the bacteriophage identified by Accession No. NITE BP-04033;
(4) a bacteriophage having lytic activity against *Pseudomonas aeruginosa,* wherein a genome of the bacteriophage comprises the nucleic acid sequence of the genome of the bacteriophage identified by Accession No. NITE BP-04036;
(5) a bacteriophage having lytic activity against *Pseudomonas aeruginosa,* wherein a genome of the bacteriophage comprises the nucleic acid sequence of the genome of the bacteriophage identified by Accession No. NITE BP-04034;
(6) a bacteriophage having lytic activity against *Pseudomonas aeruginosa,* wherein a genome of the bacteriophage comprises the nucleic acid sequence of the genome of the bacteriophage identified by Accession No. NITE BP-04035;
(7) a bacteriophage having lytic activity against *Pseudomonas aeruginosa,* wherein a genome of the bacteriophage comprises the nucleic acid sequence of the genome of the bacteriophage identified by Receipt No. NITE ABP-04200; and
(8) a bacteriophage having lytic activity against *Pseudomonas aeruginosa,* wherein a genome of the bacteriophage comprises the nucleic acid sequence of the genome of the bacteriophage identified by Receipt No. NITE ABP-04201.

In one embodiment, the bacteriophage of the present invention may include the following bacteriophages:
(1) a bacteriophage, wherein a genome of the bacteriophage consists of the nucleic acid sequence of the genome of the bacteriophage identified by Accession No. NITE BP-04040;
(2) a bacteriophage, wherein a genome of the bacteriophage consists of the nucleic acid sequence of the genome of the bacteriophage identified by Accession No. NITE BP-04031;
(3) a bacteriophage, wherein a genome of the bacteriophage consists of the nucleic acid sequence of the genome of the bacteriophage identified by Accession No. NITE BP-04033;
(4) a bacteriophage, wherein a genome of the bacteriophage consists of the nucleic acid sequence of the genome of the bacteriophage identified by Accession No. NITE BP-04036;
(5) a bacteriophage, wherein a genome of the bacteriophage consists of the nucleic acid sequence of the genome of the bacteriophage identified by Accession No. NITE BP-04034;
(6) a bacteriophage, wherein a genome of the bacteriophage consists of the nucleic acid sequence of the genome of the bacteriophage identified by Accession No. NITE BP-04035;
(7) a bacteriophage, wherein a genome of the bacteriophage consists of the nucleic acid sequence of the genome of the bacteriophage identified by Receipt No. NITE ABP-04200; and
(8) a bacteriophage, wherein a genome of the bacteriophage consists of the nucleic acid sequence of the genome of the bacteriophage identified by Receipt No. NITE ABP-04201.

The bacteriophage of the present invention may include the following bacteriophages:
(1) the bacteriophage identified by Accession No. NITE BP-04040 or a passage strain thereof;
(2) the bacteriophage identified by Accession No. NITE BP-04031 or a passage strain thereof;
(3) the bacteriophage identified by Accession No. NITE BP-04033 or a passage strain thereof;
(4) the bacteriophage identified by Accession No. NITE BP-04036 or a passage strain thereof;
(5) the bacteriophage identified by Accession No. NITE BP-04034 or a passage strain thereof;
(6) the bacteriophage identified by Accession No. NITE BP-04035 or a passage strain thereof;
(7) the bacteriophage identified by Receipt No. NITE ABP-04200 or a passage strain thereof; and
(8) the bacteriophage identified by Receipt No. NITE ABP-04201 or a passage strain thereof.

The bacteriophage of the present invention may include the following bacteriophages:
(1) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04040 by deletion, substitution, insertion, or addition of 1 to 5,000 (e.g., 1 to 1,000, 1 to 500, or 1 to 100) nucleotides or a combination thereof;
(2) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04031 by deletion, substitution, insertion, or addition of 1 to 5,000 (e.g., 1 to 1,000, 1 to 500, or 1 to 100) nucleotides or a combination thereof;
(3) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04033 by deletion, substitution, insertion, or addition of 1 to 5,000 (e.g., 1 to 1,000, 1 to 500, or 1 to 100) nucleotides or a combination thereof;
(4) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04036 by deletion, substitution, insertion, or addition of 1 to 5,000 (e.g., 1 to 1,000, 1 to 500, or 1 to 100) nucleotides or a combination thereof;
(5) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04034 by deletion, substitution, insertion, or addition of 1 to 5,000 (e.g., 1 to 1,000, 1 to 500, or 1 to 100) nucleotides or a combination thereof;
(6) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04035 by deletion, substitution, insertion, or addition of 1 to 5,000 (e.g., 1 to 1,000, 1 to 500, or 1 to 100) nucleotides or a combination thereof;
(7) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Receipt No. NITE ABP-04200 by deletion, substitution, insertion, or addition of 1 to 5,000 (e.g., 1 to 1,000, 1 to 500, or 1 to 100) nucleotides or a combination thereof; and
(8) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Receipt No. NITE ABP-04201 by deletion, substitution, insertion, or addition of 1 to 5,000 (e.g., 1 to 1,000, 1 to 500, or 1 to 100) nucleotides or a combination thereof.

For the modifications such as deletion, substitution, insertion, or addition of a nucleotide(s), a plurality of modifications may be continuous, or a plurality of modifications may exist at different positions. Such a modified form of the bacteriophage is encompassed within the bacteriophage of the present invention as long as it has lytic activity against bacteria of *Pseudomonas,* such as *Pseudomonas aeruginosa.*

During passage culture, production, and/or replication of the bacteriophage, bacteriophage mutants in which the nucleic acid sequences of the genomes contained in the bacteriophage are partially deleted, substituted, inserted, and/or added may occur. Such mutants may be included in the bacteriophage of the present invention, as long as they have lytic activity against bacteria of *Pseudomonas,* such as *Pseudomonas aeruginosa.*

The bacteriophage of the present invention also includes a passage strain of a bacteriophage identified by above-described Accession No. or Receipt No., as long as it has lytic activity against bacteria of *Pseudomonas,* such as *Pseudomonas aeruginosa.*

As used herein, the term "passage strain" means a bacteriophage obtained by passage culture of a distributed bacteriophage.

The present invention may also encompass a polynucleotide consisting of the genome contained in the bacteriophage of the present invention (hereinafter also referred to as "genome of the present invention"). Accordingly, the present invention provides:
(1) a polynucleotide of a bacteriophage genome comprising a nucleic acid sequence having 90% or more identity to the nucleic acid sequence of the genome of the bacteriophage identified by Accession No. NITE BP-04040;
(2) a polynucleotide of a bacteriophage genome comprising a nucleic acid sequence having 90% or more identity to the nucleic acid sequence of the genome of the bacteriophage identified by Accession No. NITE BP-04031;
(3) a polynucleotide of a bacteriophage genome comprising a nucleic acid sequence having 90% or more identity to the nucleic acid sequence of the genome of the bacteriophage identified by Accession No. NITE BP-04033;
(4) a polynucleotide of a bacteriophage genome comprising a nucleic acid sequence having 90% or more identity to the nucleic acid sequence of the genome of the bacteriophage identified by Accession No. NITE BP-04036;
(5) a polynucleotide of a bacteriophage genome comprising a nucleic acid sequence having 90% or more identity to the nucleic acid sequence of the genome of the bacteriophage identified by Accession No. NITE BP-04034;
(6) a polynucleotide of a bacteriophage genome comprising a nucleic acid sequence having 90% or more identity to the nucleic acid sequence of the genome of the bacteriophage identified by Accession No. NITE BP-04035;
(7) a polynucleotide of a bacteriophage genome comprising a nucleic acid sequence having 90% or more identity to the nucleic acid sequence of the genome of the bacteriophage identified by Receipt No. NITE ABP-04200; and
(8) a polynucleotide of a bacteriophage genome comprising a nucleic acid sequence having 90% or more identity to the nucleic acid sequence of the genome of the bacteriophage identified by Receipt No. NITE ABP-04201.

In one embodiment, the polynucleotide may be a polynucleotide encoding a bacteriophage having lytic activity against bacteria of *Pseudomonas,* such as *Pseudomonas aeruginosa.*

In one embodiment, the genome of the present invention may comprise the following:
(1) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Accession No. NITE BP-04040;
(2) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Accession No. NITE BP-04031;
(3) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Accession No. NITE BP-04033;
(4) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Accession No. NITE BP-04036;
(5) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Accession No. NITE BP-04034;
(6) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Accession No. NITE BP-04035;
(7) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Receipt No. NITE ABP-04200; and
(8) a polynucleotide of a bacteriophage genome comprising a nucleic acid sequence having 90% or more, 95% or more, 96% or more, 97% or more, 98% or more, or 99% or more identity to the nucleic acid sequence of the genome of the bacteriophage identified by Receipt No. NITE ABP-04201.

In one embodiment, the genome of the present invention may include the following genomes:
(1) a genome comprising the nucleic acid sequence of the genome of the bacteriophage identified by Accession No. NITE BP-04040;
(2) a genome comprising the nucleic acid sequence of the genome of the bacteriophage identified by Accession No. NITE BP-04031;
(3) a genome comprising the nucleic acid sequence of the genome of the bacteriophage identified by Accession No. NITE BP-04033;
(4) a genome comprising the nucleic acid sequence of the genome of the bacteriophage identified by Accession No. NITE BP-04036;
(5) a genome comprising the nucleic acid sequence of the genome of the bacteriophage identified by Accession No. NITE BP-04034;
(6) a genome comprising the nucleic acid sequence of the genome of the bacteriophage identified by Accession No. NITE BP-04035;
(7) a genome comprising the nucleic acid sequence of the genome of the bacteriophage identified by Receipt No. NITE ABP-04200; and
(8) a genome comprising the nucleic acid sequence of the genome of the bacteriophage identified by Receipt No. NITE ABP-04201.

The genome of the present invention may include the following genomes:
(1) a genome consisting of the nucleic acid sequence of the genome of the bacteriophage identified by Accession No. NITE BP-04040;
(2) a genome consisting of the nucleic acid sequence of the genome of the bacteriophage identified by Accession No. NITE BP-04031;
(3) a genome consisting of the nucleic acid sequence of the genome of the bacteriophage identified by Accession No. NITE BP-04033;
(4) a genome consisting of the nucleic acid sequence of the genome of the bacteriophage identified by Accession No. NITE BP-04036;
(5) a genome consisting of the nucleic acid sequence of the genome of the bacteriophage identified by Accession No. NITE BP-04034;
(6) a genome consisting of the nucleic acid sequence of the genome of the bacteriophage identified by Accession No. NITE BP-04035;
(7) a genome consisting of the nucleic acid sequence of the genome of the bacteriophage identified by Receipt No. NITE ABP-04200; and
(8) a genome consisting of the nucleic acid sequence of the genome of the bacteriophage identified by Receipt No. NITE ABP-04201.

The genome of the present invention can be produced using conventional techniques known in the art, such as recombinant DNA technology (e.g., polymerase chain reaction (PCR) amplification, cloning), enzymatic or chemical synthesis, or a combination thereof. For example, the genome of the present invention can be produced by ligating a plurality of polynucleotides containing the partial nucleotide sequences of the genome of the present invention by genetic engineering techniques. In one embodiment, the full-length of or partial sequence of the genome of the present invention may be contained in a vector known in the art.

The bacteriophage of the present invention can be obtained by making a request for distribution to the depository center.

Further, the bacteriophage of the present invention can be produced based on the sequence information by sequencing the nucleic acid sequence of the genome of the distributed bacteriophage by general techniques known in the art. For example, the genome of the present invention produced by the above method may be introduced into a host bacterium by electroporation (e.g., in the case of obtaining a bacteriophage having lytic activity against *Pseudomonas aeruginosa, Pseudomonas aeruginosa* may be used as the host bacterium). Then, the bacterium into which the genome has been introduced may be added to a plate overlaid with soft agar and cultured. Thereafter, single lytic plaques may be obtained by plaque assay. The bacteriophage of the present invention can be produced by adding the single lytic plaques to the host bacterial culture solution, followed by culturing and filtering the culture supernatant obtained by standing or centrifugation.

The bacteriophage of the present invention can be prepared by conventional culture, isolation, and purification methods known in the art. For example, host bacteria (such as bacteria of *Pseudomonas,* such as *Pseudomonas aeruginosa*) may be pre-cultured, infected with the bacteriophage of the present invention, and cultured at 37°C. After culturing, the culture supernatant obtained by standing or centrifugation can be filtered, to obtain a purified bacteriophage. The medium can be appropriately selected depending on the bacterium to be used. For example, in the case of culturing *Pseudomonas aeruginosa,* LB medium can be used. In the case of preparing a plurality of bacteriophages, they may be grown in separate host bacteria, or they may be grown in the same host bacterium.

Further, the bacteriophage of the present invention can be stored in various forms (such as in liquid and freeze-dried forms) by an appropriate method known in the art.

### < 2. Modified bacteriophage of the present invention >

In another aspect, the present invention provides a modified bacteriophage (hereinafter also referred to as the "modified bacteriophage of the present invention"). The modified bacteriophage of the present invention contains:
[1] modification of the tail fiber gene; and/or
[2] introduction of glycoside hydrolase and/or bacteriocin.

A bacteriophage to be modified is not especially limited as long as it is a bacteriophage having lytic activity against bacteria of *Pseudomonas,* such as *Pseudomonas aeruginosa.* In one embodiment, a bacteriophage to be modified may be a bacteriophage having lytic activity against bacteria of *Pseudomonas* that would cause or be responsible for infection, such as *Pseudomonas aeruginosa.*

For example, the modified bacteriophage of the present invention may be modified from the bacteriophage according to any of (1) to (12) below:
(1) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04040;
(2) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04031;
(3) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04033;
(4) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04036;
(5) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04034;
(6) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04035;
(7) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04032;
(8) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04037;
(9) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04039;
(10) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04038;
(11) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04200; and
(12) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04201.

The bacteriophage PAi23 strain was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04032).

The bacteriophage φBrkr strain was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04037).

The bacteriophage φ30-1 strain was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04039).

The bacteriophage φBrmt strain was sent for international deposition to the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the applicant and accepted on December 7, 2023 (Accession No. NITE BP-04038).

In one embodiment, the modified bacteriophage of the present invention may be a bacteriophage modified from the bacteriophage according to any of (1) to (12) below:
(1) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04040;
(2) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04031;
(3) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04033;
(4) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04036;
(5) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04034;
(6) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04035;
(7) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04032;
(8) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04037;
(9) a bacteriophage having lytic activity against bacteria of *Pseudomonas,* such as *Pseudomonas aeruginosa*, wherein a genome of the bacteriophage comprises the nucleic acid sequence of the genome of the bacteriophage identified by Accession No. NITE BP-04039;
(10) a bacteriophage having lytic activity against bacteria of *Pseudomonas*, such as *Pseudomonas aeruginosa*, wherein a genome of the bacteriophage comprises the nucleic acid sequence of the genome of the bacteriophage identified by Accession No. NITE BP-04038;
(11) a bacteriophage having lytic activity against bacteria of *Pseudomonas*, such as *Pseudomonas aeruginosa*, wherein a genome of the bacteriophage comprises the nucleic acid sequence of the genome of the bacteriophage identified by Receipt No. NITE ABP-04200; and
(12) a bacteriophage having lytic activity against bacteria of *Pseudomonas*, such as *Pseudomonas aeruginosa*, wherein a genome of the bacteriophage comprises the nucleic acid sequence of the genome of the bacteriophage identified by Receipt No. NITE ABP-04201.

In one embodiment, the modified bacteriophage of the present invention may be a bacteriophage modified from the bacteriophage according to any of (1) to (12) below:
(1) a bacteriophage, wherein a genome of the bacteriophage consists of the nucleic acid sequence of the genome of the bacteriophage identified by Accession No. NITE BP-04040;
(2) a bacteriophage, wherein a genome of the bacteriophage consists of the nucleic acid sequence of the genome of the bacteriophage identified by Accession No. NITE BP-04031;
(3) a bacteriophage, wherein a genome of the bacteriophage consists of the nucleic acid sequence of the genome of the bacteriophage identified by Accession No. NITE BP-04033;
(4) a bacteriophage, wherein a genome of the bacteriophage consists of the nucleic acid sequence of the genome of the bacteriophage identified by Accession No. NITE BP-04036;
(5) a bacteriophage, wherein a genome of the bacteriophage consists of the nucleic acid sequence of the genome of the bacteriophage identified by Accession No. NITE BP-04034;
(6) a bacteriophage, wherein a genome of the bacteriophage consists of the nucleic acid sequence of the genome of the bacteriophage identified by Accession No. NITE BP-04035;
(7) a bacteriophage, wherein a genome of the bacteriophage consists of the nucleic acid sequence of the genome of the bacteriophage identified by Accession No. NITE BP-04032;
(8) a bacteriophage, wherein a genome of the bacteriophage consists of the nucleic acid sequence of the genome of the bacteriophage identified by Accession No. NITE BP-04037;
(9) a bacteriophage, wherein a genome of the bacteriophage consists of the nucleic acid sequence of the genome of the bacteriophage identified by Accession No. NITE BP-04039;
(10) a bacteriophage, wherein a genome of the bacteriophage consists of the nucleic acid sequence of the genome of the bacteriophage identified by Accession No. NITE BP-04038;
(11) a bacteriophage, wherein a genome of the bacteriophage consists of the nucleic acid sequence of the genome of the bacteriophage identified by Receipt No. NITE ABP-04200; and
(12) a bacteriophage, wherein a genome of the bacteriophage consists of the nucleic acid sequence of the genome of the bacteriophage identified by Receipt No. NITE ABP-04201.

A bacteriophage to be modified also includes a passage strain of a bacteriophage identified by above-described Accession No. or Receipt No., as long as it has lytic activity against bacteria of *Pseudomonas*, such as *Pseudomonas aeruginosa.*

In one embodiment, the modified bacteriophage of the present invention may be a bacteriophage modified from the bacteriophage according to any of (1) to (12) below:
(1) the bacteriophage identified by Accession No. NITE BP-04040 or a passage strain thereof;
(2) the bacteriophage identified by Accession No. NITE BP-04031 or a passage strain thereof;
(3) the bacteriophage identified by Accession No. NITE BP-04033 or a passage strain thereof;
(4) the bacteriophage identified by Accession No. NITE BP-04036 or a passage strain thereof;
(5) the bacteriophage identified by Accession No. NITE BP-04034 or a passage strain thereof;
(6) the bacteriophage identified by Accession No. NITE BP-04035 or a passage strain thereof;
(7) the bacteriophage identified by Accession No. NITE BP-04032 or a passage strain thereof;
(8) the bacteriophage identified by Accession No. NITE BP-04037 or a passage strain thereof;
(9) the bacteriophage identified by Accession No. NITE BP-04039 or a passage strain thereof;
(10) the bacteriophage identified by Accession No. NITE BP-04038 or a passage strain thereof;
(11) the bacteriophage identified by Receipt No. NITE ABP-04200 or a passage strain thereof; and
(12) the bacteriophage identified by Receipt No. NITE ABP-04201 or a passage strain thereof.

Among bacteriophages to be modified, the bacteriophage identified by Accession No. or Receipt No. can be obtained by making a request for distribution to the depository center.

Bacteriophages to be modified can be prepared and cultured in the same manner as in the section < 1. Bacteriophage of the present invention > above.

### [1] Modification of tail fiber gene

In one aspect, the present invention relates to a bacteriophage having lytic activity against *Pseudomonas aeruginosa*, wherein a tail fiber gene of the bacteriophage has been modified.

The tail fiber gene and the tail fiber protein of a bacteriophage are associated with a host range that the bacteriophage infects (the host spectrum) and host specificity of the bacteriophage (WO 2016/055585 A and WO 2017/174810 A). The term "host range" or "host specificity" refers to a range of bacterial species or strains that the bacteriophage can infect. By modifying the tail fiber gene of the bacteriophage, it is possible to alter or expand the host range or host specificity of the bacteriophage. For example, the tail fiber gene of the bacteriophage may be exchanged with a tail fiber gene derived from another bacteriophage, so that the bacteriophage can acquire the host range or host specificity of another bacteriophage. Concerning the host range or host specificity, lytic activity of a bacteriophage against a particular bacterial species or strain of *Pseudomonas* (e.g., *Pseudomonas aeruginosa*) may be inspected. Thus, the host range of the particular bacterial species or strain or the host specificity to such bacterial species or strain may be determined.

In one embodiment, the modified bacteriophage of the present invention may be a bacteriophage having lytic activity against *Pseudomonas aeruginosa,* wherein a tail fiber gene of the bacteriophage has been substituted with a tail fiber gene derived from another bacteriophage having lytic activity against *Pseudomonas aeruginosa.*

Another bacteriophage having lytic activity against *Pseudomonas aeruginosa* is not particularly limited, provided that such bacteriophage is different from the bacteriophage to be modified. Examples of other bacteriophages include JG024, Phi33, PTP92, PTP47, LBL3, SPM-1, F8, PB1, KPP12, LMA2, SN, 14-1, NH-4, PTP93, PTP47, and C36.

The tail fiber genes and proteins of such bacteriophages have been known (e.g., WO 2016/055585 A and WO 2017/174810 A). An example thereof may be the tail fiber gene of phage JG024, the putative sequence thereof is registered under NCBI Reference Sequence NC_017674.1, and this sequence is shown in SEQ ID NO: 6. Also, a person skilled in the art would be able to isolate the full-length or a part of the tail fiber gene from a bacteriophage having lytic activity against *Pseudomonas aeruginosa*, substitute the tail fiber gene for a tail fiber gene in the bacteriophage to be modified, and confirm the lytic activity of the resulting bacteriophage against *Pseudomonas aeruginosa*, so as to screen for the tail fiber gene associated with the host range or host specificity of interest.

The tail fiber gene may be substituted entirely or partially (a hybrid gene), or the tail fiber gene may be substituted with a gene comprising a part of each of a plurality of tail fiber genes derived from a plurality of other bacteriophage strains. The tail fiber protein comprises a C-terminal receptor binding domain to bind to bacteria and an N-terminal domain to bind the C-terminal receptor binding domain to the bacteriophage body. Since the C-terminal receptor binding domain is considered to be associated with the host range or host specificity, it is preferable that at least the C-terminal receptor binding domain be substituted.

The tail fiber gene can be substituted by any gene recombination method known in the art without particular limitation. For example, the tail fiber gene of the bacteriophage can be substituted by inserting a tail fiber gene for substitution and a sequence for homologous recombination into a vector or plasmid suitable for the *Pseudomonas aeruginosa* host, introducing the vector or plasmid into the *Pseudomonas aeruginosa* host together with the bacteriophage genome to be modified, and performing homologous recombination. Alternatively, a genome comprising a nucleic acid sequence derived from the bacteriophage genome to be modified by substitution of the original tail fiber gene with a tail fiber gene for substitution may be prepared, and the resulting bacteriophage genome may be introduced into the *Pseudomonas aeruginosa* host. Thus, the modified bacteriophage can be prepared.

In one embodiment, the modified bacteriophage of the present invention may include the following bacteriophages:
(1) a bacteriophage having lytic activity against bacteria of *Pseudomonas,* such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04040 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(2) a bacteriophage having lytic activity against bacteria of *Pseudomonas*, such as *Pseudomonas aeruginosa*, wherein a genome of the bacteriophage (a) comprises a nucleic acid sequence having 90% or more identity to the nucleic acid sequence of the genome of the bacteriophage identified by Accession No. NITE BP-04031 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(3) a bacteriophage having lytic activity against bacteria of *Pseudomonas*, such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04033 except for the region of the tail fiber gene and (b) comprises a nucleic acid sequence of a tail fiber gene comprising at least a part of a tail fiber gene derived from another bacteriophage having lytic activity against *Pseudomonas aeruginosa;*
(4) a bacteriophage having lytic activity against bacteria of *Pseudomonas,* such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04036 except for the region of the tail fiber gene and (b) comprises a nucleic acid sequence of a tail fiber gene comprising at least a part of a tail fiber gene derived from another bacteriophage having lytic activity against *Pseudomonas aeruginosa;*
(5) a bacteriophage having lytic activity against bacteria of *Pseudomonas*, such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04034 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(6) a bacteriophage having lytic activity against bacteria of *Pseudomonas*, such as *Pseudomonas aeruginosa*, wherein a genome of the bacteriophage (a) comprises a nucleic acid sequence having 90% or more identity to the nucleic acid sequence of the genome of the bacteriophage identified by Accession No. NITE BP-04035 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(7) a bacteriophage having lytic activity against bacteria of *Pseudomonas*, such as *Pseudomonas aeruginosa*, wherein a genome of the bacteriophage (a) comprises a nucleic acid sequence having 90% or more identity to the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(8) a bacteriophage having lytic activity against bacteria of *Pseudomonas,* such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04037 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(9) a bacteriophage having lytic activity against bacteria of *Pseudomonas*, such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04039 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(10) a bacteriophage having lytic activity against bacteria of *Pseudomonas*, such as *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04038 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(11) a bacteriophage having lytic activity against bacteria of *Pseudomonas,* such as *Pseudomonas aeruginosa,* wherein a genome of the bacteriophage (a) comprises a nucleic acid sequence having 90% or more identity to the nucleic acid sequence of the genome of the bacteriophage identified by Receipt No. NITE ABP-04200 except for the region of the tail fiber gene and (b) comprises a nucleic acid sequence of a tail fiber gene comprising at least a part of a tail fiber gene derived from another bacteriophage having lytic activity against *Pseudomonas aeruginosa*; and
(12) a bacteriophage having lytic activity against bacteria of *Pseudomonas,* such as *Pseudomonas aeruginosa,* wherein a genome of the bacteriophage (a) comprises a nucleic acid sequence having 90% or more identity to the nucleic acid sequence of the genome of the bacteriophage identified by Receipt No. NITE ABP-04201 except for the region of the tail fiber gene and (b) comprises a nucleic acid sequence of a tail fiber gene comprising at least a part of a tail fiber gene derived from another bacteriophage having lytic activity against *Pseudomonas aeruginosa.*

In one embodiment, the modified bacteriophage of the present invention may include the following bacteriophages:
(1) a bacteriophage having lytic activity against bacteria of *Pseudomonas,* such as *Pseudomonas aeruginosa*, wherein a genome of the bacteriophage (a) comprises the nucleic acid sequence of the genome of the bacteriophage identified by Accession No. NITE BP-04040 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(2) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04031 except for the region of the tail fiber gene and (b) comprises a nucleic acid sequence of a tail fiber gene comprising at least a part of a tail fiber gene derived from another bacteriophage having lytic activity against *Pseudomonas aeruginosa;*
(3) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04033 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(4) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04036 except for the region of the tail fiber gene and (b) comprises a nucleic acid sequence of a tail fiber gene comprising at least a part of a tail fiber gene derived from another bacteriophage having lytic activity against *Pseudomonas aeruginosa;*
(5) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04034 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(6) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04035 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(7) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa;*
(8) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Accession No. NITE BP-04037 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(9) a bacteriophage having lytic activity against bacteria of *Pseudomonas*, such as *Pseudomonas aeruginosa*, wherein a genome of the bacteriophage (a) comprises the nucleic acid sequence of the genome of the bacteriophage identified by Accession No. NITE BP-04039 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(10) a bacteriophage having lytic activity against bacteria of *Pseudomonas,* such as *Pseudomonas aeruginosa*, wherein a genome of the bacteriophage (a) comprises the nucleic acid sequence of the genome of the bacteriophage identified by Accession No. NITE BP-04038 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(11) a bacteriophage having lytic activity against bacteria of *Pseudomonas*, such as *Pseudomonas aeruginosa*, wherein a genome of the bacteriophage (a) comprises the nucleic acid sequence of the genome of the bacteriophage identified by Receipt No. NITE ABP-04200 except for the region of the tail fiber gene and (b) comprises a nucleic acid sequence of a tail fiber gene comprising at least a part of a tail fiber gene derived from another bacteriophage having lytic activity against *Pseudomonas aeruginosa*; and
(12) a bacteriophage having lytic activity against bacteria of *Pseudomonas,* such as *Pseudomonas aeruginosa,* wherein a genome of the bacteriophage (a) comprises the nucleic acid sequence of the genome of the bacteriophage identified by Receipt No. NITE ABP-04201 except for the region of the tail fiber gene and (b) comprises a nucleic acid sequence of a tail fiber gene comprising at least a part of a tail fiber gene derived from another bacteriophage having lytic activity against *Pseudomonas aeruginosa.*

In one embodiment, the modified bacteriophage of the present invention may include the following bacteriophages:
(1) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04040 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(2) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04031 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(3) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04033 except for the region of the tail fiber gene and (b) comprises a nucleic acid sequence of a tail fiber gene comprising at least a part of a tail fiber gene derived from another bacteriophage having lytic activity against *Pseudomonas aeruginosa;*
(4) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04036 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(5) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04034 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(6) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04035 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(7) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(8) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04037 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(9) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04039 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(10) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Accession No. NITE BP-04038 except for the region of the tail fiber gene and (b) comprises a nucleic acid sequence of a tail fiber gene comprising at least a part of a tail fiber gene derived from another bacteriophage having lytic activity against *Pseudomonas aeruginosa*;
(11) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Receipt No. NITE ABP-04200 except for the region of the tail fiber gene and (b) comprises a nucleic acid sequence of a tail fiber gene comprising at least a part of a tail fiber gene derived from another bacteriophage having lytic activity against *Pseudomonas aeruginosa*; and
(12) a bacteriophage having lytic activity against bacteria of *Pseudomonas,* such as *Pseudomonas aeruginosa,* wherein a genome of the bacteriophage (a) consists of the nucleic acid sequence of the genome of the bacteriophage identified by Receipt No. NITE ABP-04201 except for the region of the tail fiber gene and (b) comprises a nucleic acid sequence of a tail fiber gene comprising at least a part of a tail fiber gene derived from another bacteriophage having lytic activity against *Pseudomonas aeruginosa.*

In one embodiment, the modified bacteriophage of the present invention may comprise a nucleic acid sequence of a tail fiber gene derived from another bacteriophage having lytic activity against *Pseudomonas aeruginosa.* In one embodiment, the modified bacteriophage of the present invention may comprise a nucleic acid sequence encoding the tail fiber gene derived from bacteriophage JG024. In one embodiment, a tail fiber gene derived from another bacteriophage may be the tail fiber gene of bacteriophage JG024. For example, such gene may comprise the nucleic acid sequence shown in SEQ ID NO: 6 or a nucleic acid sequence having 90% or more, 95% or more, 96% or more, 97% or more, 98% or more, or 99% or more identity to SEQ ID NO: 6. In one embodiment, the modified bacteriophage of the present invention may comprise the nucleic acid sequence shown in SEQ ID NO: 6.

In one embodiment, the modified bacteriophage of the present invention may be the bacteriophage described below:
a bacteriophage having lytic activity against *Pseudomonas aeruginosa,* wherein a genome of the bacteriophage (a) consists of the nucleic acid sequence of the genome of the bacteriophage identified by Accession No. NITE BP-04032 except for the region of the tail fiber gene and (b) comprises a nucleic acid sequence encoding the tail fiber gene derived from bacteriophage JG024.

In one embodiment, the modified bacteriophage of the present invention may be the bacteriophage described below:
a bacteriophage having lytic activity against *Pseudomonas aeruginosa,* wherein a genome of the bacteriophage (a) consists of the nucleic acid sequence of the genome of the bacteriophage identified by Accession No. NITE BP-04032 except for the region of the tail fiber gene and (b) comprises the nucleic acid sequence shown in SEQ ID NO: 6.

### [2] Introduction of glycoside hydrolase and/or bacteriocin

In one aspect, the present invention relates to a bacteriophage having lytic activity against *Pseudomonas aeruginosa* comprising glycoside hydrolase and/or bacteriocin introduced thereinto.

Glycoside hydrolase derived from *Pseudomonas aeruginosa* is a protein that inhibits formation of *Pseudomonas aeruginosa* biofilms and destroys biofilms (Science Advances, 2016; 2 (5): e1501632). By modifying the bacteriophage of the present invention through introduction of glycoside hydrolase, the modified bacteriophage can acquire the ability to degrade *Pseudomonas aeruginosa* biofilms and have the enhanced lytic activity. When antibiotics are used in combination, improved antibiotic effects can be expected. Examples of known glycoside hydrolases include PelA and PslG degrading biofilm-constituting extracellular polysaccharides Pel (a polysaccharide consisting of N-acetylgalactosamine and N-acetylglucosamine) and Psl (a polysaccharide composed of a pentasaccharide repeat unit of d-mannose, D-glucose, and L-rhamnose) and active domains thereof PelAh and PslGh. The notation "PelA" used herein refers to the PelA protein, an active domain thereof PelAh, or a fragment thereof, provided that it has Pel-degrading activity. The notation "PslG" used herein refers to the PslG protein, an active domain thereof PslGh, or a fragment thereof, provided that it has Psl-degrading activity.

PelA, PslG, and active domains thereof have been known in the art. For example, the amino acid sequence of PelA derived from the *Pseudomonas aeruginosa* PAO1 strain is registered under GenBank Accession No. AAG06452.1 (Science Advances, 2016; 2 (5): e1501632), and the nucleic acid sequence of pelA encoding PelA comprises a sequence shown in SEQ ID NO: 1. The nucleic acid sequence encoding PelAh, which is an active domain of PelA at the N terminus, comprises the sequence shown in SEQ ID NO: 3. For example, the amino acid sequence of PslG derived from the *Pseudomonas aeruginosa* PAO1 strain is registered under GenBank Accession No. AAG05625.1 (J. Biol. Chem., 2015; 290 (47): 28374-28387), and the nucleic acid sequence of pslG encoding PslG comprises a sequence shown in SEQ ID NO: 2. The nucleic acid sequence encoding PslGh, which is an active domain of PslG lacking a transmembrane domain at the N terminus of PslG, comprises the sequence shown in SEQ ID NO: 4.

In one embodiment, the modified bacteriophage of the present invention may be a bacteriophage having lytic activity against *Pseudomonas aeruginosa,* wherein a genome of the bacteriophage comprises a nucleic acid sequence encoding PelA or an active domain thereof. In one embodiment, the modified bacteriophage of the present invention may be a bacteriophage having lytic activity against *Pseudomonas aeruginosa,* wherein a genome of the bacteriophage comprises a nucleic acid sequence encoding PelA derived from *Pseudomonas aeruginosa* or an active domain thereof (e.g., PelAh). In one embodiment, the modified bacteriophage of the present invention may be a bacteriophage having lytic activity against *Pseudomonas aeruginosa,* wherein a genome of the bacteriophage comprises a nucleic acid sequence encoding PelA derived from *Pseudomonas aeruginosa* or PelAh and the nucleic acid sequence encoding PelA or PelAh comprises (a) the nucleic acid sequence shown in SEQ ID NO: 1 or 3 or (b) a nucleic acid sequence having 90% or more, 95% or more, 96% or more, 97% or more, 98% or more, or 99% or more identity to SEQ ID NO: 1 or 3 and encoding a protein having Pel-degrading activity.

In one embodiment, the modified bacteriophage of the present invention may be a bacteriophage having lytic activity against *Pseudomonas aeruginosa,* wherein a genome of the bacteriophage comprises a nucleic acid sequence encoding PslG or an active domain thereof. In one embodiment, the modified bacteriophage of the present invention may be a bacteriophage having lytic activity against *Pseudomonas aeruginosa,* wherein a genome of the bacteriophage comprises a nucleic acid sequence encoding PslG derived from *Pseudomonas aeruginosa* or an active domain thereof (e.g., PslGh). In one embodiment, the modified bacteriophage of the present invention may be a bacteriophage having lytic activity against *Pseudomonas aeruginosa,* wherein a genome of the bacteriophage comprises a nucleic acid sequence encoding PslG derived from *Pseudomonas aeruginosa* or PslGh and the nucleic acid sequence encoding PslG or PslGh comprises (a) the nucleic acid sequence shown in SEQ ID NO: 2 or 4 or (b) a nucleic acid sequence having 90% or more, 95% or more, 96% or more, 97% or more, 98% or more, or 99% or more identity to SEQ ID NO: 2 or 4 and encoding a protein having Psl-degrading activity.

Bacteriocin is a protein having antibacterial activity against similar or closely related bacteria produced by the bacteria of interest. An example of a known factor having antibacterial activity against *Pseudomonas aeruginosa* is bacteriocin derived from *Pseudomonas aeruginosa,* Pyocin G (PyoG) (Journal of Molecular Biology, 2020; 432 (13): 3869-3880). By modifying the bacteriophage of the present invention through introduction of bacteriocin, the resulting bacteriophage can acquire the enhanced antibacterial activity against *Pseudomonas aeruginosa.*

PyoG has been known in the art. For example, the amino acid sequence of PyoG derived from *Pseudomonas aeruginosa* BWHPSA046 is registered under GenBank Accession No. ETV05907.1 (Journal of Molecular Biology, 2020; 432 (13): 3869-3880), and it is encoded by a sequence shown in SEQ ID NO: 5.

In one embodiment, the modified bacteriophage of the present invention may be a bacteriophage having lytic activity against *Pseudomonas aeruginosa,* wherein a genome of the bacteriophage comprises a nucleic acid sequence encoding Pyocin G. In one embodiment, the modified bacteriophage of the present invention may be a bacteriophage having lytic activity against *Pseudomonas aeruginosa,* wherein a genome of the bacteriophage comprises a nucleic acid sequence encoding Pyocin G derived from *Pseudomonas aeruginosa.* In one embodiment, the modified bacteriophage of the present invention may be a bacteriophage having lytic activity against *Pseudomonas aeruginosa,* wherein a genome of the bacteriophage comprises a nucleic acid sequence encoding Pyocin G derived from *Pseudomonas aeruginosa* and the nucleic acid sequence encoding Pyocin G comprises (a) the nucleic acid sequence shown in SEQ ID NO: 5 or (b) a nucleic acid sequence having 90% or more, 95% or more, 96% or more, 97% or more, 98% or more, or 99% or more identity to SEQ ID NO: 5 and encoding a protein having bacteriocin activity.

The nucleic acid sequence encoding glycoside hydrolase and/or bacteriocin may be introduced into the bacteriophage to be placed under the control of an adequate regulatory sequence, so that it would be expressed as a protein in the bacteriophage. For example, the nucleic acid sequence encoding glycoside hydrolase and/or bacteriocin may be operably linked to an adequate promoter sequence, such as a promoter sequence having activity in *Pseudomonas aeruginosa* (e.g., the *Pseudomonas aeruginosa* rpsB gene promoter, the *Pseudomonas aeruginosa* fda gene promoter, or the *E. coli* lac promoter).

In one embodiment, a spacer (1 to 10 nucleotides) may be provided between a regulatory sequence (e.g., a promoter sequence) and another regulatory sequence or between a regulatory sequence and the nucleic acid sequence encoding glycoside hydrolase and/or bacteriocin. In a specific embodiment, a sequence composed of: a part of the lac operon and the Shine-Dalgarno-like sequence (SEQ ID NO: 8) as a regulatory sequence; and a nucleic acid sequence encoding PslGh, which are linked to each other (SEQ ID NO: 10) and a sequence composed of: a part of the lac operon, a spacer (5 nucleotides: TTAGA), and the Shine-Dalgarno-like sequence as a regulatory sequence; and a nucleic acid sequence encoding PslGh, which are linked to each other (SEQ ID NO: 11) may be used for the modified bacteriophage of the present invention.

Glycoside hydrolase and/or bacteriocin can be introduced into a bacteriophage by any gene recombination method known in the art without particular limitation. For example, a nucleic acid sequence encoding glycoside hydrolase and/or bacteriocin and a sequence for homologous recombination may be introduced into a vector or plasmid suitable for the *Pseudomonas aeruginosa* host, the vector or plasmid and the bacteriophage genome to be modified may be introduced into the *Pseudomonas aeruginosa* host, and homologous recombination may be performed. Thus, a nucleic acid sequence encoding the glycoside hydrolase and/or bacteriocin can be introduced into the bacteriophage. Alternatively, a nucleic acid sequence encoding glycoside hydrolase and/or bacteriocin may be inserted into the genome of the bacteriophage to be modified, and the resulting bacteriophage genome may be introduced into the *Pseudomonas aeruginosa* host. Thus, a modified bacteriophage can be prepared.

When preparing a bacteriophage comprising Pyocin G introduced thereinto as bacteriocin, it is preferable that a gene encoding an immunoprotein (ImG) produced as a multidomain protein be introduced into host bacteria used for amplification together with Pyocin G. Thus, the activity of the bacteriocin Pyocin G produced by a bacteriophage would be inhibited by the immunoprotein and host bacteria would be protected (Journal of Molecular Biology, 2020; 432 (13): 3869-3880).

In one embodiment, the modified bacteriophage of the present invention is a bacteriophage having lytic activity against *Pseudomonas aeruginosa,* wherein a genome of the bacteriophage comprises a nucleic acid sequence encoding at least one protein or active domain selected from (a) to (c) below:
(a) Pyocin G;
(b) PslG or PslGh; and
(c) PelA or PelAh.

In one embodiment, the modified bacteriophage of the present invention is a bacteriophage having lytic activity against *Pseudomonas aeruginosa,* wherein a genome of the bacteriophage comprises a nucleic acid sequence encoding at least two proteins or active domains selected from (a) to (c) below:
(a) Pyocin G;
(b) PslG or PslGh; and
(c) PelA or PelAh.

In one embodiment, the modified bacteriophage of the present invention is a bacteriophage having lytic activity against *Pseudomonas aeruginosa,* wherein a genome of the bacteriophage comprises a nucleic acid sequence encoding proteins or active domains (a) to (c) below:
(a) Pyocin G;
(b) PslG or PslGh; and
(c) PelA or PelAh.

In one embodiment, the modified bacteriophage of the present invention is a bacteriophage having lytic activity against *Pseudomonas aeruginosa,* wherein a genome of the bacteriophage comprises a nucleic acid sequence encoding at least one protein derived from *Pseudomonas aeruginosa* or active domain selected from (a) to (c) below:
(a) Pyocin G;
(b) PslG or PslGh; and
(c) PelA or PelAh.

In one embodiment, the modified bacteriophage of the present invention is a bacteriophage having lytic activity against *Pseudomonas aeruginosa,* wherein a genome of the bacteriophage comprises at least one nucleic acid sequence selected from (a) to (c) below:
(a) the nucleic acid sequence shown in SEQ ID NO: 5 or a nucleic acid sequence having 90% or more, 95% or more, 96% or more, 97% or more, 98% or more, or 99% or more identity to SEQ ID NO: 5 and encoding a protein having bacteriocin activity;
(b) the nucleic acid sequence shown in SEQ ID NO: 2 or 4 or a nucleic acid sequence having 90% or more, 95% or more, 96% or more, 97% or more, 98% or more, or 99% or more identity to SEQ ID NO: 2 or 4 and encoding a protein having Psl-degrading activity; and
(c) the nucleic acid sequence shown in SEQ ID NO: 1 or 3 or a nucleic acid sequence having 90% or more, 95% or more, 96% or more, 97% or more, 98% or more, or 99% or more identity to SEQ ID NO: 1 or 3 and encoding a protein having Pel-degrading activity.

In one embodiment, the modified bacteriophage of the present invention may include the bacteriophages below:
(1) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04040 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(2) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04031 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(3) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04033 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(4) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04036 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(5) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04034 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(6) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04035 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(7) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04032 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(8) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04037 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(9) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04039 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh; and
(10) a bacteriophage having lytic activity against bacteria of *Pseudomonas,* such as *Pseudomonas aeruginosa,* wherein a genome of the bacteriophage comprises a nucleic acid sequence having 90% or more identity to the nucleic acid sequence of the genome of the bacteriophage identified by Accession No. NITE BP-04038 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh.

In one embodiment, the modified bacteriophage of the present invention may include the bacteriophages below:
(1) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04040 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(2) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04031 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(3) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04033 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(4) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04036 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(5) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04034 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(6) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04035 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(7) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04032 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(8) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04037 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(9) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04039 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh; and
(10) a bacteriophage having lytic activity against bacteria of *Pseudomonas,* such as *Pseudomonas aeruginosa,* wherein a genome of the bacteriophage comprises a nucleic acid sequence modified from the nucleic acid sequence of the genome of the bacteriophage identified by Accession No. NITE BP-04038 by deletion, substitution, insertion, or addition of 1 to 5,000 (e.g., 1 to 1,000, 1 to 500, or 1 to 100) nucleotides or a combination thereof (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh.

For the modifications such as deletion, substitution, insertion, or addition of a nucleotide(s), a plurality of modifications may be continuous, or a plurality of modifications may exist at different positions.

In one embodiment, the modified bacteriophage of the present invention may include the bacteriophages below:
(1) a bacteriophage having lytic activity against bacteria of *Pseudomonas,* such as *Pseudomonas aeruginosa,* wherein a genome of the bacteriophage comprise the nucleic acid sequence of the genome of the bacteriophage identified by Accession No. NITE BP-04040 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(2) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04031 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(3) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04033 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(4) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04036 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(5) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04034 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(6) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04035 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(7) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04032 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh
(8) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04037 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh;
(9) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04039 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa;* and/or
   (B) a nucleic acid sequence encoding at least one protein or active domain selected from (a) to (c) below:
      (a) Pyocin G;
      (b) PslG or PslGh; and
      (c) PelA or PelAh; and
(10) a bacteriophage having lytic activity against bacteria of *Pseudomonas,* such as *Pseudomonas aeruginosa,* wherein a genome of the bacteriophage comprises the nucleic acid sequence of the genome of the bacteriophage identified by Accession No. NITE BP-04038 (except for the region of the tail fiber gene in the case of (A) below) and comprises a nucleic acid sequence (A) and/or a nucleic acid sequence (B) below:
   (A) a nucleic acid sequence of a tail fiber gene comprising at least a part of a tail fiber gene derived from another bacteriophage having lytic activity against *Pseudomonas aeruginosa*; and/or
   (B) a nucleic acid sequence encoding at least one protein or active domain selected from (a) to (c) below:
      (a) Pyocin G;
      (b) PslG or PslGh; and
      (c) PelA or PelAh.

In one embodiment, the modified bacteriophage of the present invention may include the bacteriophage below:
a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04032 except for the region of the tail fiber gene and comprises a nucleic acid sequence (A) and/or a nucleic acid sequence (B) below:
(A) a nucleic acid sequence of a tail fiber gene comprising at least a part of the tail fiber gene derived from bacteriophage JG024, and/or
(B) a nucleic acid sequence encoding at least one protein or active domain selected from (a) to (c) below:
   (a) Pyocin G;
   (b) PslG or PslGh; and
   (c) PelA or PelAh.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 and a nucleic acid sequence encoding at least one protein or active domain derived from *Pseudomonas aeruginosa* selected from (a) to (c) below:
(a) Pyocin G;
(b) PslG or PslGh; and
(c) PelA or PelAh.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 and at least one nucleic acid sequence selected from (a) to (c) below:
(a) the nucleic acid sequence shown in SEQ ID NO: 5 or a nucleic acid sequence having 90% or more, 95% or more, 96% or more, 97% or more, 98% or more, or 99% or more identity to SEQ ID NO: 5 and encoding a protein having bacteriocin activity;
(b) the nucleic acid sequence shown in SEQ ID NO: 2 or 4 or a nucleic acid sequence having 90% or more, 95% or more, 96% or more, 97% or more, 98% or more, or 99% or more identity to SEQ ID NO: 2 or 4 and encoding a protein having Psl-degrading activity; and
(c) the nucleic acid sequence shown in SEQ ID NO: 1 or 3 or a nucleic acid sequence having 90% or more, 95% or more, 96% or more, 97% or more, 98% or more, or 99% or more identity to SEQ ID NO: 1 or 3 and encoding a protein having Pel-degrading activity.

In one embodiment, the modified bacteriophage of the present invention may include the bacteriophage below:
a bacteriophage having lytic activity against *Pseudomonas aeruginosa,* wherein a genome of the bacteriophage comprises a nucleic acid sequence having 90% or more identity to the genome of the bacteriophage identified by Accession No. NITE BP-04032 except for the region of the tail fiber gene and comprises nucleic acid sequences (A) and (B) below:
(A) a nucleic acid sequence encoding the tail fiber gene derived from bacteriophage JG024; and
(B) a nucleic acid sequence encoding at least one protein or active domain derived from *Pseudomonas aeruginosa* selected from (a) to (c) below:
   (a) Pyocin G;
   (b) PslG or PslGh; and
   (c) PelA or PelAh.

In one embodiment, the modified bacteriophage of the present invention may include the bacteriophage below:
a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04032 except for the region of the tail fiber gene and comprises nucleic acid sequences (A) and (B) below:
(A) a nucleic acid sequence encoding the tail fiber gene derived from bacteriophage JG024; and
(B) a nucleic acid sequence encoding PslGh.

In one embodiment, the modified bacteriophage of the present invention may include the bacteriophage below:
a bacteriophage having lytic activity against *Pseudomonas aeruginosa,* wherein a genome of the bacteriophage comprises a nucleic acid sequence having 90% or more identity to the genome of the bacteriophage identified by Accession No. NITE BP-04032 except for the region of the tail fiber gene and comprises nucleic acid sequences (A) and (B) below:
(A) a nucleic acid sequence encoding the tail fiber gene derived from bacteriophage JG024; and
(B) a nucleic acid sequence encoding PslGh and PelAh.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) a nucleic acid sequence encoding the tail fiber gene derived from bacteriophage JG024; and
(B) a nucleic acid sequence encoding at least one protein or active domain derived from *Pseudomonas aeruginosa* selected from (a) to (c) below:
   (a) Pyocin G;
   (b) PslG or PslGh; and
   (c) PelA or PelAh.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) a nucleic acid sequence encoding the tail fiber gene derived from bacteriophage JG024; and
(B) at least one nucleic acid sequence selected from (a) to (c) below:
   (a) the nucleic acid sequence shown in SEQ ID NO: 5 or a nucleic acid sequence having 90% or more, 95% or more, 96% or more, 97% or more, 98% or more, or 99% or more identity to SEQ ID NO: 5 and encoding a protein having bacteriocin activity;
   (b) the nucleic acid sequence shown in SEQ ID NO: 2 or 4 or a nucleic acid sequence having 90% or more, 95% or more, 96% or more, 97% or more, 98% or more, or 99% or more identity to SEQ ID NO: 2 or 4 and encoding a protein having Psl-degrading activity; and
   (c) the nucleic acid sequence shown in SEQ ID NO: 1 or 3 or a nucleic acid sequence having 90% or more, 95% or more, 96% or more, 97% or more, 98% or more, or 99% or more identity to SEQ ID NO: 1 or 3 and encoding a protein having Pel-degrading activity.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6 or a nucleic acid sequence having 90% or more identity to SEQ ID NO: 6; and
(B) at least one nucleic acid sequence selected from (a) to (c) below,
   (a) the nucleic acid sequence shown in SEQ ID NO: 5 or a nucleic acid sequence having 90% or more identity to SEQ ID NO: 5 and encoding a protein having bacteriocin activity;
   (b) the nucleic acid sequence shown in SEQ ID NO: 2 or 4 or a nucleic acid sequence having 90% or more identity to SEQ ID NO: 2 or 4 and encoding a protein having Psl-degrading activity; and
   (c) the nucleic acid sequence shown in SEQ ID NO: 1 or 3 or a nucleic acid sequence having 90% or more identity to SEQ ID NO: 1 or 3 and encoding a protein having Pel-degrading activity.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6 or a nucleic acid sequence having 90% or more identity to SEQ ID NO: 6; and
(B) the nucleic acid sequence shown in SEQ ID NO: 4 or a nucleic acid sequence having 90% or more identity to SEQ ID NO: 4 and encoding a protein having Psl-degrading activity.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6 or a nucleic acid sequence having 90% or more identity to SEQ ID NO: 6; and
(B) the nucleic acid sequence shown in SEQ ID NO: 4 or a nucleic acid sequence having 90% or more identity to SEQ ID NO: 4 and encoding a protein having Psl-degrading activity, and
   the nucleic acid sequence shown in SEQ ID NO: 3 or a nucleic acid sequence having 90% or more identity to SEQ ID NO: 3 and encoding a protein having Pel-degrading activity.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6; and
(B) at least one nucleic acid sequence selected from (a) to (c) below,
   (a) the nucleic acid sequence shown in SEQ ID NO: 5;
   (b) the nucleic acid sequence shown in SEQ ID NO: 2 or 4; and
   (c) the nucleic acid sequence shown in SEQ ID NO: 1 or 3.

In one embodiment, the modified bacteriophage of the present invention may include the bacteriophage below:
a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6; and
(B) the nucleic acid sequence shown in SEQ ID NO: 4, SEQ ID NO: 10, or SEQ ID NO: 11.

### < 3. Uses of pharmaceutical composition and bacteriophage of the present invention >

The bacteriophage of the present invention and/or the modified bacteriophage of the present invention have lytic activity against bacteria of *Pseudomonas,* particularly *Pseudomonas aeruginosa,* which cause infection. Therefore, the bacteriophage of the present invention and/or the modified bacteriophage of the present invention are expected to be effective for treating or preventing infection.

**In** another aspect, the present invention provides uses of the bacteriophage of the present invention and/or the modified bacteriophage of the present invention, for example, a pharmaceutical composition comprising, as an active component, the bacteriophage of the present invention and/or the modified bacteriophage of the present invention (hereinafter also referred to as "the pharmaceutical composition of the present invention").

The pharmaceutical composition of the present invention may include a pharmaceutical composition comprising at least one bacteriophage including the bacteriophage of the present invention and/or the modified bacteriophage of the present invention, and a pharmaceutically acceptable excipient.

The pharmaceutical composition of the present invention may further comprise one or more strains of a bacteriophage known to have lytic activity against bacteria of *Pseudomonas,* such as *Pseudomonas aeruginosa.* Examples of such a strain include, but are not particularly limited to, the bacteriophage of the present invention, the modified bacteriophage of the present invention, and other known bacteriophages.

The pharmaceutical composition of the present invention can be prepared using an excipient conventionally used in the art, that is, a pharmaceutical excipient, a pharmaceutical carrier, and the like by a conventional method. Examples of dosage forms of such a pharmaceutical composition may include parenteral formulations such as injections, infusions, powder inhalers, and nebulizers, and it can be administered by intravenous administration, transpulmonary administration, or the like. For formulation, excipients, carriers, additives, or the like can be used depending on these dosage forms within a pharmaceutically acceptable range. For example, the pharmaceutical composition of the present invention can be produced by mixing the bacteriophage with a pharmaceutically acceptable excipient or suspending the bacteriophage in a pharmaceutically acceptable excipient.

The pharmaceutical composition of the present invention may include a pharmaceutical composition comprising at least two types of bacteriophages and a pharmaceutically acceptable excipient.

The pharmaceutical composition of the present invention may include a pharmaceutical composition comprising three types of bacteriophages and a pharmaceutically acceptable excipient.

The pharmaceutical composition of the present invention may include a pharmaceutical composition comprising four types of bacteriophages and a pharmaceutically acceptable excipient.

The pharmaceutical composition of the present invention may include a pharmaceutical composition comprising five types of bacteriophages and a pharmaceutically acceptable excipient.

The pharmaceutical composition of the present invention can comprise bacteriophages of any types in combination. In one embodiment, the pharmaceutical composition of the present invention may comprise bacteriophages with different properties in combination. Examples of bacteriophages include pili-recognizing phages and LPS-recognizing phages.

Examples of pili-recognizing phages include, but are not limited to, the bacteriophage identified by Accession No. NITE BP-04040, the bacteriophage identified by Receipt No. NITE ABP-04200, the bacteriophage identified by Accession No. NITE BP-04037, the bacteriophage identified by Receipt No. NITE ABP-04201, the bacteriophage identified by Accession No. NITE BP-04039, and the bacteriophage identified by Accession No. NITE BP-04036, and passage strains thereof.

Examples of LPS-recognizing phages include, but are not limited to: the bacteriophage identified by Accession No. NITE BP-04031; a bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and encodes the nucleic acid sequences (A) and (B): (A) a nucleic acid sequence encoding the tail fiber gene derived from bacteriophage JG024, and (B) a nucleic acid sequence encoding PslGh; the bacteriophage identified by Accession No. NITE BP-04033; and passage strains thereof.

The pharmaceutical composition of the present invention may comprise at least one type of bacteriophage selected from a group of pili-recognizing phages and at least one type of bacteriophage selected from a group of LPS-recognizing phages.

In one embodiment, the pharmaceutical composition of the present invention may include a pharmaceutical composition comprising the bacteriophages (i) and (ii) below and a pharmaceutically acceptable excipient:
(i) the bacteriophage identified by Accession No. NITE BP-04037 or a passage strain thereof; and
(ii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 4 or SEQ ID NO: 10.

In one embodiment, the pharmaceutical composition of the present invention may include a pharmaceutical composition comprising the bacteriophages (i) and (ii) below and a pharmaceutically acceptable excipient:
(i) the bacteriophage identified by Accession No. NITE BP-04037 or a passage strain thereof; and
(ii) the bacteriophage identified by Accession No. NITE BP-04031 or a passage strain thereof.

In one embodiment, the pharmaceutical composition of the present invention may include a pharmaceutical composition comprising the bacteriophages (i) and (ii) below and a pharmaceutically acceptable excipient:
(i) the bacteriophage identified by Accession No. NITE BP-04040 or a passage strain thereof; and
(ii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 4 or SEQ ID NO: 10.

In one embodiment, the pharmaceutical composition of the present invention may include a pharmaceutical composition comprising the bacteriophages (i) and (ii) below and a pharmaceutically acceptable excipient:
(i) the bacteriophage identified by Accession No. NITE BP-04040 or a passage strain thereof; and
(ii) the bacteriophage identified by Accession No. NITE BP-04031 or a passage strain thereof.

In one embodiment, the pharmaceutical composition of the present invention may include a pharmaceutical composition comprising the bacteriophages (i) and (ii) below and a pharmaceutically acceptable excipient:
(i) the bacteriophage identified by Accession No. NITE BP-04039 or a passage strain thereof; and
(ii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 4 or SEQ ID NO: 10.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04037 or Receipt No. NITE ABP-04201 or a passage strain thereof;
(ii) the bacteriophage identified by Accession No. NITE BP-04031 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 10 or SEQ ID NO: 11; and
(iv) the bacteriophage identified by Accession No. NITE BP-04040 or Receipt No. NITE ABP-04200.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04037 or a passage strain thereof;
(ii) the bacteriophage identified by Accession No. NITE BP-04040 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 4 or SEQ ID NO: 10; and
(iv) the bacteriophage identified by Accession No. NITE BP-04031 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04037 or a passage strain thereof;
(ii) the bacteriophage identified by Receipt No. NITE ABP-04200 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11; and
(iv) the bacteriophage identified by Accession No. NITE BP-04031 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Receipt No. NITE ABP-04201 or a passage strain thereof;
(ii) the bacteriophage identified by Receipt No. NITE ABP-04200 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11; and
(iv) the bacteriophage identified by Accession No. NITE BP-04031 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04037 or a passage strain thereof;
(ii) the bacteriophage identified by Receipt No. NITE ABP-04200 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11; and
(iv) the bacteriophage identified by Accession No. NITE BP-04033 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04037 or a passage strain thereof;
(ii) the bacteriophage identified by Accession No. NITE BP-04036 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11; and
(iv) the bacteriophage identified by Accession No. NITE BP-04031 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04039 or a passage strain thereof;
(ii) the bacteriophage identified by Receipt No. NITE ABP-04200 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11; and
(iv) the bacteriophage identified by Accession No. NITE BP-04031 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Accession No. NITE BP-04039 or a passage strain thereof;
(ii) the bacteriophage identified by Accession No. NITE BP-04036 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11; and
(iv) the bacteriophage identified by Accession No. NITE BP-04033 or a passage strain thereof.

In one embodiment, the pharmaceutical composition of the present invention may include a pharmaceutical composition comprising the bacteriophages (i) to (iv) below and a pharmaceutically acceptable excipient:
(i) the bacteriophage identified by Receipt No. NITE ABP-04200 or a passage strain thereof;
(ii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
   (A) the nucleic acid sequence shown in SEQ ID NO: 6; and
   (B) the nucleic acid sequence shown in SEQ ID NO: 11;
(iii) the bacteriophage identified by Accession No. NITE BP-04031 or a passage strain thereof; and
(iv) the bacteriophage identified by Accession No. NITE BP-04033 or a passage strain thereof.

The effective dose, the administration frequency, and the administration period may vary depending on the purpose of administration (therapeutic or preventive purpose), the severity and age of the subject to which the bacteriophage is administered, the dosage form of the preparation to be used, the potency of the bacteriophage, and the like. For example, about 10⁴ to 10¹⁴ plaque forming unit (PFU) can be used as an effective dose of a single bacteriophage or as a total effective dose of two or more bacteriophages. The dosage ratio of the two or more bacteriophages can be appropriately adjusted according to the severity of symptoms and age of the patient, the dosage form of the preparation to be used, the potency of the bacteriophage, or the like. In the case where the pharmaceutical composition comprises three types of bacteriophages, for example, each bacteriophage may be contained in approximately equal amounts (e.g., 3.3 × 10³ to 3.3 × 10¹³ PFU), or each bacteriophage may be contained in different ratio. In the case where the pharmaceutical composition comprises four types of bacteriophages, for example, each bacteriophage may be contained in approximately equal amounts (e.g., 2.5 × 10³ to 2.5 × 10¹³ PFU), or each bacteriophage may be contained in different ratio.

The pharmaceutical composition of the present invention can be used as an agent for preventing or treating infection with bacteria of *Pseudomonas,* such as infection with *Pseudomonas aeruginosa.* The term "infection" used herein refers to infection caused by infection with bacteria of *Pseudomonas,* such as *Pseudomonas aeruginosa,* or infection for which such bacteria are responsible. Examples thereof include lung, respiratory tract, skin, subcutaneous tissue, bone, ear, eye, urinary tract, cardiac valve, blood, and systemic infections. In the case of lung or respiratory tract infection, examples of infection symptoms include, but are not limited to, coughing, sputum, bloody sputum, fever, dyspnea, malaise, pulmonary nodules, and bronchiectasis. In the case of skin, subcutaneous tissue, bone, ear, eye, or urinary tract infection, examples of infection symptoms include, but are not limited to, pus discharge, fever, and inflammation. In the case of blood or systemic infection, examples of symptoms include, but are not limited to, bacillemia, ichorrhemia, septic shock, fever, blood pressure drop, and anuria.

As used herein, "treat," "treating," and "treatment" mean at least partial improvement of infection symptoms, cessation of progress or worsening of infection, complete cure, and the like. As used herein, "prevent," "preventing," and "prevention" mean preventing a subject not suffering from infection from developing infection, preventing recurrence of infection, and the like.

The present invention may encompass a pharmaceutical composition for preventing or treating infection caused by bacteria of *Pseudomonas,* such as infection with *Pseudomonas aeruginosa,* comprising the bacteriophage or the modified bacteriophage of the present invention or the pharmaceutical composition of the present invention. In one embodiment, infection with *Pseudomonas aeruginosa* may be respiratory infection. In one embodiment, infection with *Pseudomonas aeruginosa* may be lung infection.

Further, the present invention may encompass a method for preventing or treating infection caused by bacteria of *Pseudomonas,* such as infection with *Pseudomonas aeruginosa,* in a subject. Such method comprises a step of administering a therapeutically effective amount of the bacteriophage or the modified bacteriophage of the present invention or the pharmaceutical composition of the present invention. In one embodiment, at least two, for example, three, four, or five types of bacteriophages or modified bacteriophages of the present invention may be administered to the subject, and the plurality of bacteriophages may be administered simultaneously or separately.

Further, the present invention encompasses the bacteriophage or the modified bacteriophage of the present invention or the pharmaceutical composition of the present invention for use in preventing or treating infection caused by bacteria of *Pseudomonas,* such as infection with *Pseudomonas aeruginosa.* Further, the present invention encompasses use of the bacteriophage or the modified bacteriophage of the present invention or the pharmaceutical composition of the present invention in the manufacture of a pharmaceutical composition for preventing or treating infection caused by bacteria of *Pseudomonas,* such as infection with *Pseudomonas aeruginosa.*

According to the present invention, the subject to which the bacteriophage is administered may not be limited, as long as it is a mammal, and examples thereof may include a mouse, a rat, a dog, a pig, a monkey, and a human. For example, the bacteriophage may be administered to a subject diagnosed with infection with bacteria of *Pseudomonas,* such as *Pseudomonas aeruginosa,* or a subject at risk of suffering from infection with bacteria of *Pseudomonas,* such as *Pseudomonas aeruginosa* (e.g., a hospitalized patient, an immunodeficient patient, or an externally injured patient).

The bacteriophage or the modified bacteriophage of the present invention or the pharmaceutical composition of the present invention may also be used or administered in combination with other active agents effective for treating or preventing infection with bacteria of *Pseudomonas,* such as *Pseudomonas aeruginosa,* and the bacteriophage or the modified bacteriophage of the present invention or the pharmaceutical composition of the present invention may be provided as a combination therapy with such other active agents. Examples of active agents include, but are not limited to, antibiotics, such as fluoroquinolone, carbapenem, aminoglycoside, ansamycin, cephalosporin, penicillin, β-lactam, β-lactamase inhibitor, folate pathway inhibitor, fusidane, glycopeptide, glycylcycline, lincosamide, lipopeptide, macrolide, quinolone, oxazolidinone, phenicols phosphonic acid, streptogramin, tetracycline, sulfonamide, imipenem, meropenem, amikacin, ciprofloxacin, levofloxacin, tobramycin, azithromycin, aztreonam, and colistin.

### < 4. Use of prophage-lacking strain as host bacterium >

The present invention also relates to use of a strain lacking a prophage-derived gene from *Pseudomonas aeruginosa* as a host bacterium used for production of a bacteriophage having lytic activity against *Pseudomonas aeruginosa.* In one aspect, accordingly, the present invention provides a method for producing a bacteriophage having lytic activity against *Pseudomonas aeruginosa,* the method comprising a step of culturing the bacteriophage in a host bacterium from which a prophage-derived gene has been deleted.

According to the present invention, the term "prophage" refers to a lysogenic bacteriophage that is inserted into the genome of a host bacterium *(Pseudomonas aeruginosa)* and lysogenized therein. A single type of host bacterium may contain a single type of prophage or two or more types of prophages. By deleting a prophage-derived gene to remove the prophage from *Pseudomonas aeruginosa,* the resulting *Pseudomonas aeruginosa* can be used as a host bacterium to produce a bacteriophage with high efficiency.

The term "prophage-derived gene" used herein refers to a gene that is present in a prophage, and, in particular, the term refers to a gene associated with maintenance of a prophage. An example of a "prophage-derived gene" is the pflM gene (Journal of Bacteriology, 2024; 206 (5): e0040223).

When "lacking a prophage-derived gene" or "a prophage-derived gene is deleted" herein, 1) the full-length of at least one gene is deleted from the genome sequence of a prophage, 2-1) the genome sequence of a prophage may be modified by deletion, substitution, insertion, or addition of a part of at least one gene, or a combination thereof to lack the prophage from a host bacterium, or 2-2) functions of the prophage-derived gene are lacked. The prophage-derived gene can be deleted by a method known in the art in accordance with a type of a host bacterium used and a type of a gene to be deleted (e.g., Journal of Bacteriology, 2024; 206 (5): e0040223).

According to the method of the present invention, a target bacteriophage; i.e., a bacteriophage having lytic activity against *Pseudomonas aeruginosa,* may be inoculated into the host bacterium lacking a prophage-derived gene, or the genome of the bacteriophage may be introduced into the host bacterium, and culture may then be performed. Culture conditions may adequately be selected in accordance with a host bacterium and a method of culture employed. Following culture, the culture supernatant obtained by standing or centrifugation may be filtered, so that a purified bacteriophage can be obtained.

The present invention has been generally described above, but specific examples are now provided for reference for further understanding. These examples are for illustrative purposes and are not intended to limit the invention.

### Examples

Unless otherwise specified, the experiment was conducted according to the attached protocol for the parts using commercially available kits or reagents. Moreover, concentration mol/L is represented as M for convenience of description. For example, a 1M sodium hydroxide aqueous solution means a 1 mol/L sodium hydroxide aqueous solution.

Strains indicated by ATCC can be obtained from American Type Culture Collection and strains indicated by NBRC can be obtained from National Institute of Technology and Evaluation (NITE). The PA14 strain (Catalog No. NR-50573), the MRSN 321 strain (Catalog No. NR-51517), and the MRSN 4841 strain can be obtained from BEI Resources, NIAID, NIH. The MRSN 321 strain and the MRSN 4841 strain are contained in the *Pseudomonas aeruginosa* Diversity Panel provided by Multidrug-Resistant Organism Repository and Surveillance Network (MRSN) at the Walter Reed Army Institute of Research (WRAIR).

### < Example 1: Acquisition of bacteriophage exhibiting lytic activity against Pseudomonas aeruginosa >

Bacteriophages were isolated from environment samples (sewage water or discharged water in Japan) in the manner described below. For bacteriophage isolation, any of the *Pseudomonas aeruginosa* (Schroeter) Migula ATCC15692 strain (an allied strain of the PAO1 strain), NBRC3080 strain, PA14 strain, MRSN 321 strain, or MRSN 4841 strain was used as the host strain. The environment sample was centrifuged, the supernatant was collected, and the supernatant was then filtered through a 0.22 µm or 0.45 µm filter. The filtered sample was mixed with an equivalent amount of LB liquid medium adjusted to be two-fold of the final concentration (20 g/L LB medium, Lennox (Nacalai Tesque, Inc.)). To the mixture, a culture solution of *Pseudomonas aeruginosa* that had been cultured overnight in LB liquid medium was added and cultured with shaking at 37°C. The culture solution was centrifuged, and the supernatant was collected. The supernatant and the culture solution of *Pseudomonas aeruginosa* that had been cultured overnight in LB liquid medium were mixed with LB liquid medium containing 0.3% to 0.6% soft agar, and the mixture was overlaid on an LB agar plate. Culture was performed at 37°C for several hours to several days, lytic plaques appeared on the medium were collected, and bacteriophages were isolated. The bacteriophages obtained are referred to as PAPT1, PAi140, PAi228, PAi239, and PAi242, respectively. Lytic plaques were collected from the bacteriophage stock distributed from Rakuno Gakuen University in the same manner as described above, and bacteriophages φLCX and φLCX2 (closely related strains of the bacteriophage φLP identified by NCBI Reference Sequence LC727700) were isolated. These bacteriophages were cultured in soft agar-containing LB liquid medium in the same manner as described above using the *Pseudomonas aeruginosa* ATCC15692 strain, NBRC3080 strain, or NBRC13746 strain as the host strain to prepare bacteriophage lysates.

The bacteriophage PAi23 strain distributed from Gifu University (a passage strain of PaGU11 identified by NCBI Reference Sequence NC_050145) and the bacteriophage φBrkr strain (a passage strain of the bacteriophage identified by NCBI Reference Sequence LC765218), the bacteriophage φBrmt strain (a passage strain of the bacteriophage identified by NCBI Reference Sequence LC727695), and the bacteriophage φ30-1 strain (a passage strain of the bacteriophage identified by NCBI Reference Sequence LC727696) distributed from Rakuno Gakuen University were cultured with the *Pseudomonas aeruginosa* ATCC15692 strain to prepare bacteriophage lysates.

The lytic activity of PAPT1, PAi140, PAi228, PAi239, PAi242, φLCX, φLCX2, PAi23, φBrkr, φBrmt, and φ30-1 *against Pseudomonas aeruginosa* was examined in the manner described below. A culture solution of *Pseudomonas aeruginosa* that had been cultured overnight in LB liquid medium was mixed with LB liquid medium containing 0.3% or 0.6% soft agar, and the mixture was overlaid on an LB agar plate. The 10-fold serially diluted solutions of the bacteriophage lysates prepared using the phage buffer (10 mM Tris-HCl (pH7.5) containing 68.4 mM NaCl, 1 mM CaCl₂, 10 mM MgSO₄) were added dropwise to the plate in at 2.5 µL each and subjected to static culture at 37°C. Lytic plaques of the bacteriophages appeared, and the lytic activity thereof against *Pseudomonas aeruginosa* was confirmed (Fig. 1).

Accession Nos. or Receipt Nos. of PAPT1, PAi140, PAi228, PAi239, PAi242, PAi23, φBrkr, φBrmt, φ30-1, φLCX, and φLCX2 obtained above are Accession No. NITE BP-04031, Accession No. NITE BP-04033, Accession No. NITE BP-04034, Accession No. NITE BP-04035, Accession No. NITE BP-04036, Accession No. NITE BP-04032, Accession No. NITE BP-04037, Accession No. NITE BP-04038, Accession No. NITE BP-04039, Accession No. NITE BP-04040, and Receipt No. NITE ABP-04200, respectively.

### < Example 2: Construction of tail-fiber-gene-exchanged bacteriophage >

The tail fiber gene of the PAi23 phage was exchanged with the tail fiber gene of the known *Pseudomonas aeruginosa* bacteriophage JG024 strain (the bacteriophage identified by NCBI Reference Sequence NC_017674.1) to construct a recombinant bacteriophage. A plurality of polynucleotides each comprising the PAi23 phage genome and a partial nucleotide sequence of the tail fiber gene of the artificially synthesized JG024 phage (SEQ ID NO: 6) were amplified by PCR. The amplified polynucleotides were linked to each other using the Gibson assembly master mix (Catalog No. E2611, New England Biolabs). As a result, a recombinant phage genome lacking nucleotides 31081 to 33975 encoding the tail fiber gene of the PAi23 phage and comprising the tail fiber gene of the JG024 phage (SEQ ID NO: 6) integrated thereinto was constructed. The phage genome obtained was introduced into the *Pseudomonas aeruginosa* ATCC15692 strain via electroporation to obtain a bacteriophage of interest. The bacteriophage obtained is referred to as "PAi23-JG024 tail."

The lytic activity of PAi23-JG024 tail obtained above against *Pseudomonas aeruginosa* was examined. The *Pseudomonas aeruginosa* ATCC15692 strain and the respiratory clinical isolate 3-55-PA strain and 9-8-PA strain (distributed by Three Academic Societies Joint Antimicrobial Susceptibility Surveillance Program) were used. A culture solution of *Pseudomonas aeruginosa* that had been cultured overnight in LB liquid medium was mixed with LB liquid medium containing 0.6% soft agar, and the resulting mixture was overlaid on an LB agar plate. Using the phage buffer, 10-fold serially diluted solutions of the PAi23 phage and PAi23-JG024 tail phage lysates were prepared. The diluted solutions (2.5 µL each) were added dropwise to the plate and subjected to static culture at 37°C. Lytic plaques of the bacteriophages appeared, and the lytic activity thereof against *Pseudomonas aeruginosa* was confirmed (Fig. 2). As shown in Table 1, PAi23-JG024 tail was found to infect the strains that would not be infected with PAi23.

**[Table 1]**

| *Pseudomonas aeruginosa* | Phage | |
|---|---|---|
| | PAi23 | PAi23-JG024 tail |
| ATCC15692 | + | + |
| 3-55-PA | - | + |
| 9-8-PA | - | + |

| | | |
|---|---|---|
| +: Lytic activity was observed; -: No lytic activity was observed. | | |

### < Example 3: Construction of PAi23-JG024 tail phage carrying PelAh, PslGh, and PslG >

The PelAh gene (SEQ ID NO: 3), the PslGh gene (SEQ ID NO: 4), and the PslG gene (SEQ ID NO: 2) derived from *Pseudomonas aeruginosa* were inserted into the PAi23 phage genome to construct phages comprising such genes inserted thereinto. PelAh is a fragment of the PelA gene (SEQ ID NO: 1), and PslGh is a fragment of the PslG gene (SEQ ID NO: 2) (Science advances, 2016; 2 (5): e1501632). The sequences of the PelAh gene, the PslGh gene, and the PslG gene were amplified by PCR from the genome of the NBRC106052(PAO1) strain. The ampicillin resistant gene of the pUCP18 plasmid vector (NCBI Reference Sequence U07164) was substituted with a gentamycin resistant gene to obtain a pUCP18-GmR plasmid vector. The Shine-Dalgarno-like sequence (SEQ ID NO: 8) was added to a region closer to the 5' end of the initiation codon of the PelAh gene, the PslGh gene, and the PslG gene, respectively, and the resultants were inserted into the *Eco*RI/*Xba*I restriction enzyme site to be linked to the lac operon of the pUCP18-GmR plasmid vector. In the same manner as in Example 2, the PelAh gene, the PslGh gene, and the PslG gene, to each of which a part of the lac operon (SEQ ID NO: 7) and the Shine-Dalgarno-like sequence (SEQ ID NO: 8) were added, were each inserted into a site between nucleotide 12081 and nucleotide 12082 of the PAi23 phage genome (a sequence comprising a part of the lac operon, the Shine-Dalgarno-like sequence (SEQ ID NO: 8), and the PslGh gene linked to each other is SEQ ID NO: 10). In the same manner as in Example 2, in addition, a phage genome comprising a tail fiber gene substituted with the tail fiber gene of the JG024 phage was constructed. The constructed phage genomes were introduced into the *Pseudomonas aeruginosa* ATCC15692 strain via electroporation to obtain gene recombinant bacteriophages, and the resultants are referred to as PAi23-JG024 tail::Plac-PelAh (AB), PAi23-JG024 tail::Plac-PslGh (AB), and PAi23-JG024 tail::Plac-PslG (AB), respectively.

In the same manner as described above, the Shine-Dalgarno-like sequence (SEQ ID NO: 8), which is a non-coding sequence located 5' to the initiation codon, was substituted with the Shine-Dalgarno-like sequence _version 3(v3) comprising a spacer added thereto (SEQ ID NO: 12) to construct a phage. The PslGh gene (SEQ ID NO: 4), to which a part of the lac operon (SEQ ID NO: 7) and the Shine-Dalgarno-like sequence _v3 (SEQ ID NO: 12) were added, was inserted into a site between nucleotide 12081 and nucleotide 12082 of the PAi23 phage genome (a sequence comprising a part of the lac operon, the Shine-Dalgarno-like sequence _v3 (SEQ ID NO: 12), and the PslGh gene linked to each other is SEQ ID NO: 11). In the same manner as in Example 2, in addition, a phage genome comprising a tail fiber gene substituted with the tail fiber gene of the JG024 phage was constructed. The constructed phage genome was introduced into the *Pseudomonas aeruginosa* ATCC15692 strain via electroporation to obtain a gene recombinant bacteriophage, and the resultant is referred to as PAi23-JG024 tail::Plac-PslGh (AB)_v3.

In addition, a phage comprising the PslGh gene (SEQ ID NO: 4) and the PelAh gene (SEQ ID NO: 3) inserted thereinto was constructed. A nucleotide sequence composed of: the PslGh gene (SEQ ID NO: 4), to which a part of the lac operon (SEQ ID NO: 7) and the Shine-Dalgarno-like sequence _v3 (SEQ ID NO: 12) were added, linked to the PelAh gene (SEQ ID NO: 3), to which the Shine-Dalgarno-like sequence (SEQ ID NO: 8) was added, was inserted into a site between nucleotide 12081 and nucleotide 12082 of the PAi23 phage genome. In the same manner as in Example 2, in addition, a phage genome comprising a tail fiber gene substituted with the tail fiber gene of the JG024 phage was constructed. The constructed phage genome was introduced into the *Pseudomonas aeruginosa* ATCC15692 strain via electroporation to obtain a gene recombinant bacteriophage, and the resultant is referred to as PAi23-JG024 tail::Plac-PslGh-PelAh(AB)_v3.

The lytic activity of PAi23-JG024 tail::Plac-PelAh (AB), PAi23-JG024 tail::Plac-PslGh (AB), PAi23-JG024 tail::Plac-PslG (AB), PAi23-JG024 tail::Plac-PslGh (AB)_v3, and PAi23-JG024 tail::Plac-PslGh-PelAh (AB)_v3 against *Pseudomonas aeruginosa* was examined. The *Pseudomonas aeruginosa* ATCC15692 strain was used. A culture solution of *Pseudomonas aeruginosa* that had been cultured overnight in LB liquid medium was mixed with LB liquid medium containing 0.3% soft agar, and the resulting mixture was overlaid on an LB agar plate. Using the phage buffer, 10-fold serially diluted solutions of the phage lysates were prepared. The diluted solutions (2.5 µL each) were added dropwise to the plate and subjected to static culture at 37°C. Lytic plaques of the bacteriophages appeared, and the lytic activity thereof against *Pseudomonas aeruginosa* was confirmed (Fig. 3).

### < Example 4: Construction of PAi23 phage carrying PyoG >

The PyoG gene derived from *Pseudomonas aeruginosa* was inserted into the PAi23 phage genome to construct a bacteriophage. Bacteriocin PyoG has antibacterial activity against *Pseudomonas aeruginosa.* In order to stably produce a bacteriophage comprising the PyoG gene inserted thereinto, the *Pseudomonas aeruginosa* ATCC15692 strain that would constitutively express the ImG gene (Journal of Molecular Biology, 2020; 432 (13): 3869-3880) known as the PyoG immunity gene was constructed in advance. The ImG gene (SEQ ID NO: 9) to which the Shine-Dalgarno-like sequence (SEQ ID NO: 8) was added was inserted into the *Eco*RI/*Xba*I site of the pUCP18-GmR plasmid vector and the resultant was introduced into the *Pseudomonas aeruginosa* ATCC15692 strain via electroporation. Thus, the *Pseudomonas aeruginosa* ATCC15692-ImG strain that would constitutively express the ImG gene under the control of the lac promoter was obtained.

In the same manner as described in Example 2, subsequently, a phage genome comprising the artificially synthesized PyoG gene (SEQ ID NO: 5) inserted into a site between nucleotide 35068 and nucleotide 35069 of the PAi23 phage genome was constructed. A gene recombinant bacteriophage obtained by introducing the resulting phage genome into the *Pseudomonas aeruginosa* ATCC15692-ImG strain is referred to as PAi23::PyoG (CD).

The lytic activity of PAi23::PyoG (CD) obtained above against *Pseudomonas aeruginosa* was examined in the manner described below. The *Pseudomonas aeruginosa* ATCC15692 strain and ATCC15692-ImG strain were used. A culture solution of *Pseudomonas aeruginosa* that had been cultured overnight in LB liquid medium was mixed with LB liquid medium containing 0.3% soft agar, and the resulting mixture was overlaid on an LB agar plate. Using the phage buffer, 10-fold serially diluted solutions of PAi23 and PAi23::PyoG (CD) phage lysates were prepared. The diluted solutions (2.5 µL each) were added dropwise to the plate and subjected to static culture at 37°C. Lytic plaques of the bacteriophages appeared, and the lytic activity thereof against *Pseudomonas aeruginosa* was confirmed (Fig. 4). In the *Pseudomonas aeruginosa* ATCC15692 strain, PAi23::PyoG (CD) was found to form lytic plaques clearer than those formed by the PAi23 phage.

### < Example 5: Biofilm-degrading activity of naturally-occurring bacteriophage >

Whether or not a naturally-occurring bacteriophage has activity of reducing the amount of biofilms formed by *Pseudomonas aeruginosa* was examined. The *Pseudomonas aeruginosa* ATCC15692 strain was inoculated into a 96-well plate (Nunc, Thermo Scientific) containing LB liquid medium and the plate was covered with a lid with pins (Nunc Immuno TSP Lids, Thermo Scientific). Static culture was performed at 37°C for 24 hours to form a biofilm on the pins. The formed biofilm was washed with physiological saline, and a phage buffer, phage φLCX adjusted to the final concentration of 1.29 × 10⁸ PFU/mL with LB liquid medium, and PAPT1 or PAi239 adjusted to the final concentration of 1 × 10⁸ PFU/mL with LB liquid medium were brought into contact with the biofilm on the 96-well plate at 37°C for 6 hours. Each well was washed with physiological saline and subjected to crystal violet staining. After staining, each well was washed with physiological saline. Thereafter, crystal violet was eluted from each well with the aid of ethanol, and the absorbance of each eluate at 590 nm was measured. A higher absorbance indicates a larger biofilm amount. The experiment was performed triplicate (per condition) and repeated three times (3 trials). The average of the 3 trials was determined and one-way analysis of variance and the Dunnett's multiple comparison test were performed. The results demonstrate that the addition of φLCX, PAPT1, or PAi239 results in a significant reduction in the biofilm amount compared with the addition of a phage buffer (a control group) (Fig. 5, *p < 0.05).

### < Example 6: Biofilm-degrading activity of modified bacteriophage >

Whether or not PAi23-JG024 tail::Plac-PslGh (AB) and PAi23-JG024 tail::Plac-PslG (AB) have activity of reducing the amount of biofilms formed by *Pseudomonas aeruginosa* was examined. In the same manner as in Example 5, PAi23-JG024 tail, PAi23-JG024 tail::Plac-PslGh (AB), and PAi23-JG024 tail::Plac-PslG (AB) were each added to LB liquid medium to the final concentration of 1 × 10⁸ PFU/mL and brought into contact with the biofilms on the 96-well plate. The experiment was performed in 6 wells per condition and repeated three times (3 trials). The average of the 3 trials was determined and one-way analysis of variance and the Dunnett's multiple comparison test were performed. The results demonstrate that PAi23-JG024 tail did not reduce the biofilm amount but PAi23-JG024 tail::Plac-PslGh (AB) ("::PslGh" in Fig. 6) and PAi23-JG024 tail::Plac-PslG (AB) ("::PslG" in Fig. 6) reduced the biofilm amount to a significant extent (Fig. 6A, ^{*}p < 0.05).

Whether or not PAi23-JG024 tail::Plac-PelAh (AB) has activity of reducing the amount of biofilms formed by *Pseudomonas aeruginosa* was then examined. In the same manner as described above, biofilms were formed on pins using the respiratory clinical isolate 28-42-PA strain (distributed by Three Academic Societies Joint Antimicrobial Susceptibility Surveillance Program). PAi23-JG024 tail and PAi23-JG024 tail::Plac-PelAh (AB) were each added to LB liquid medium to the final concentration of 1 × 10⁸ PFU/mL and brought into contact with the biofilms on the 96-well plate. The experiment was performed in triplicate per condition and repeated eight times (8 trials) (the left graph in Fig. 6B). The biofilm amount relative to the biofilm amount of the control group was calculated based on the biofilm amount of the control group considered as 1 in each trial, and the Kruskal-Wallis test and the Dunn's multiple comparison test were performed. The results demonstrate that PAi23-JG024 tail did not reduce the biofilm amount but PAi23-JG024 tail::Plac-PelAh (AB) ("::PelAh" in Fig. 6) reduced the biofilm amount to a significant extent (Fig. 6B, right, ^{*}p < 0.05).

Whether or not PAi23-JG024 tail::Plac-PslGh (AB)_v3 and PAi23-JG024 tail::Plac-PslGh-PelAh (AB)_v3 have activity of reducing the amount of biofilms formed by *Pseudomonas aeruginosa* was then examined. In the same manner as described above, biofilms were formed on pins using the *Pseudomonas aeruginosa* ATCC15692 strain. PAi23-JG024 tail, PAi23-JG024 tail::Plac-PslGh (AB)_v3, and PAi23-JG024 tail::Plac-PslGh-PelAh (AB)_v3 were each added to LB liquid medium to the final concentration of 1 × 10⁸ PFU/mL and brought into contact with the biofilms on the 96-well plate. The experiment was performed in triplicate per condition and repeated three times (3 trials). The average of the 3 trials was determined and one-way analysis of variance and the Dunnett's multiple comparison test were performed. The results demonstrate that PAi23-JG024 tail did not reduce the biofilm amount but PAi23-JG024 tail::Plac-PslGh (AB)_v3 ("::PslGh _v3" in Fig. 6) and PAi23-JG024 tail::Plac-PslGh-PelAh (AB)_v3 ("::PslGh-PelAh_v3" in Fig. 6) reduced the biofilm amount to a significant extent (Fig. 6C, ^{*}p < 0.05).

### < Example 7: Inhibitory activity of modified bacteriophage on Pseudomonas aeruginosa growth >

Inhibitory activity of PAi23 and PAi23::PyoG (CD) on the growth of *Pseudomonas aeruginosa* was examined. The respiratory clinical isolate 27-12-PA strain (distributed from Three Academic Societies Joint Antimicrobial Susceptibility Surveillance Program) was diluted with LB liquid medium to OD600 of 0.1 and inoculated at 90 µL/well into a 96-well plate. Phage buffer (the control) or PAi23 and PAi23::PyoG (CD) were added at 10 µL/well to the final concentration of 1 × 10⁸ PFU/mL and static culture was performed at 37°C. The culture solution was subjected to measurement of the absorbance at 600 nm using a plate reader with the elapse of time. The experiment was performed in triplicate per condition and repeated four times (4 trials). The average absorbance of the 4 trials was calculated 12 hours after the initiation of the experiment and one-way analysis of variance and the Tukey's multiple comparisons were performed. The results demonstrate that PAi23::PyoG (CD) exerts the effects of inhibiting *Pseudomonas aeruginosa* growth for a longer period of time in comparison with PAi23 (Fig. 7, ^{*}p < 0.05).

### < Example 8: Lytic activity of bacteriophage φLCX against Pseudomonas aeruginosa >

Lytic activity of bacteriophage φLCX against *Pseudomonas aeruginosa* that had been cultured overnight and sufficiently grown was examined. The *Pseudomonas aeruginosa* ATCC15692 strain was inoculated into LB liquid medium and cultured with shaking in glass test tubes at 37°C. The culture solution was subjected to measurement of the absorbance at 600 nm 15 hours after the initiation of culture and measurement was performed with the elapse of time. Phage buffer (the control) or bacteriophage φLCX was added to the final concentration of 1 × 10⁷ PFU/mL 18 hours after the initiation of culture and culture with shaking was continued at 37°C. The experiment was performed in three glass test tubes under one conditions. The average absorbance of the three test tubes was determined. The results demonstrate that φLCX has lytic activity against *Pseudomonas aeruginosa* after the logarithmic growth phase (Fig. 8).

### < Example 9: Lytic activity of single bacteriophage in infected mouse model >

The *Pseudomonas aeruginosa* ATCC15692 strain was inoculated into Tryptic Soy Broth (Merck) and cultured with shaking at 37°C for 2.5 hours. The culture solution was centrifuged, the supernatant was discarded, and the resultant was suspended in physiological saline. The suspension was diluted with physiological saline to adjust the absorbance at 600 nm (OD600) to 0.5 to prepare an inoculum. C57BL/6Jms Slc mice (female, about 5-week-old, Japan SLC, Inc.) were infected via intranasal administration of 40 µL of the inoculum per mouse. Phage buffer, PAi23, or φBrmt phage was administered intranasally to groups each consisting of 5 mice at 5 × 10⁸ PFU/mouse 30 minutes later. The lungs were extracted 24 hours after infection and homogenized in 2 ml of the Hank's balanced salt solution (Life Technologies Corporation) to prepare a tissue homogenate. The tissue homogenate was diluted with physiological saline to prepare 10-fold serially diluted solution. The resultant was applied to a tryptic soy agar medium (Eiken Chemical Co., Ltd.) and the viable bacterial count was determined. After the viable bacterial count was converted to a common logarithm, the arithmetic mean was determined, and one-way analysis of variance and the Dunnett's multiple comparison test were performed. Fig. 9 shows the viable bacterial counts in the lungs 24 hours after infection (log CFU/lung) (N = 5, mean ± standard error). The results demonstrate that administration of PAi23 or φBrmt results in the viable bacterial count in the lung significantly lower than that achieved by administration of the phage buffer (a control group) (Fig. 9, ^{*}p < 0.05).

### < Example 10: Preparation of freeze-thaw resistant φBrkr phage >

In the manner described below, the φBrkr freeze-thaw resistant strain was prepared using φBrkr (Accession No. NITE BP-04037) as a parent phage and was referred to as "φBrkr_FTR2".

In a phage buffer, ethyl methanesulfonate (Nacalai Tesque, Inc.) was added to φBrkr to the final concentration of 60 mM, and the resulting mixture was reacted at 37°C for 1 hour. In the same manner as in Example 1, a lysate of bacteriophages that had been induced to genetically mutate after the reaction was prepared. The prepared lysate was diluted to 20-fold using phage buffer. Thereafter, the resultant was subjected to a step of freezing in a freezer at -80°C and thawing (a step of freeze-thaw) two times. This step of freeze-thaw was performed two times. Thereafter, the remnant bacteriophages were amplified and the lysate was prepared again in the same manner as in Example 1. The above-described procedure was defined as one cycle, and 9 cycles were performed. The lysate obtained after the 9 cycles was cultured by the same procedure as described above, and φBrkr_FTR2 purified from a single plaque was obtained. Receipt No. for φBrkr_FTR2 is NITE ABP-04201.

φBrkr_FTR2 was confirmed to have acquired freeze-thaw resistance by the following method. φBrkr and φBrkr_FTR2 were each diluted to 1 × 10⁸ PFU/mL with a phage buffer and then subjected to freeze-thaw. The titer values before and after freeze-thaw were determined by the following method. The culture solution of *Pseudomonas aeruginosa* that had been cultured overnight in LB liquid medium was mixed with LB liquid medium containing 0.3% soft agar, and the resulting mixture was overlaid on an LB agar plate. Using the phage buffer, 10-fold serially diluted solutions of the bacteriophage solutions were prepared. The diluted solutions (2.5 µL each) were added dropwise to the plate and subjected to static culture at 37°C. Lytic plaques of the bacteriophages appeared, and the lytic activity thereof against *Pseudomonas aeruginosa* was confirmed (Fig. 10A). The titer values were calculated based on the number of lytic plaques, and the rates of phage collection before and after freeze-thaw were calculated (Fig. 10B). As a result, it was confirmed that the freeze-thaw resistance of φBrkr_FTR2 was improved to a significant extent in comparison with that of φBrkr (unpaired t-test, ^{*}p < 0.05).

### < Example 11: Extension of effects of inhibiting Pseudomonas aeruginosa growth by preparing bacteriophage cocktail >

In the present example and subsequent examples, ATCC15692ΔPf4/Pf6 (a prophage-lacking strain) described in Example 14 below was used as the culture host for PAPT1, PAi242, φBrkr, φBrkr_FTR2, φ30-1, φLCX, φLCX2, PAi23-JG024 tail::Plac-PslGh (AB), and PAi23-JG024 tail::Plac-PslGh (AB)_v3 to prepare bacteriophage lysates.

Whether or not a bacteriophage cocktail would extend the effects of inhibiting *Pseudomonas aeruginosa* growth was examined. The *Pseudomonas aeruginosa* ATCC15692 strain was inoculated into a 96-well plate containing LB liquid medium to a concentration of approximately 10⁸ PFU/mL. In addition, the bacteriophage solution was added to each well to bring the amount of the solution to 100 µL in total. A single type of bacteriophage, a cocktail of two types of bacteriophages, and a cocktail of four types of bacteriophages were each added to the wells to the final concentration of 1 × 10⁴ PFU/mL (i.e., multiplicity of infection (MOI) of approximately 0.0001). A bacteriophage cocktail was prepared by mixing a plurality of types of bacteriophages at an equal ratio. In the case of a cocktail of four types of bacteriophages, for example, four types of bacteriophages were added to the final concentration of 0.25 × 10⁴ PFU/mL each. Culture was performed at 37°C and the absorbance at 600 nm in each well was measured every hour.

Fig. 11 shows representative examples of *Pseudomonas aeruginosa* growth curves: i.e., a graph demonstrating growth curves of Cocktail a (a cocktail of φBrkr and PAi23-JG024 tail::Plac-PslGh (AB)) and of each of bacteriophages constituting Cocktail a; and a graph demonstrating growth curves of Cocktail A (a cocktail of φBrkr, φLCX, PAi23-JG024 tail::Plac-PslGh (AB), and PAPT1) and of each of bacteriophages constituting Cocktail A. Similar graphs were prepared concerning Cocktail a to Cocktail e and Cocktail A to Cocktail H, and the area under the curve (AUC) of each thereof was calculated. AUC calculated without the addition of phages was considered to be 100% and AUC(%) ± standard error relative thereto was calculated. The results of calculation are shown in Table 2 and Table 3 below. As a result of one-way analysis of variance and the Tukey's multiple comparison test, all the cocktails were found to extend the effects of inhibiting *Pseudomonas aeruginosa* growth compared with each bacteriophage constituting the cocktails to a significant extent (^{*}p < 0.05).

**[Table 2]**

| | Phage | φBrkr | PAi23-JG024 tail ::Plac-PslGh (AB) | Cocktail a |
|---|---|---|---|---|
| Cocktail a | Relative AUC (%) | 55.1 ± 4.7 | 43.3 ± 1.0 | 7.3 ± 0**.**4 |
| | v.s. Cocktail a | * | * | |

| | Phage | φBrkr | PAPT1 | Cocktail b |
|---|---|---|---|---|
| Cocktail b | Relative AUC (%) | 55.1 ± 4.7 | 51.6 ± 3.9 | 22.1 ± 2.3 |
| | v.s. Cocktail b | * | * | |

| | Phage | φLCX | PAi23-JG024 tail ::Plac-PslGh (AB) | Cocktail c |
|---|---|---|---|---|
| Cocktail c | Relative AUC (%) | 70.9 ± 7.8 | 43.3 ± **1.0** | 20.2 ± 2.1 |
| | v.s. Cocktail c | * | * | |

| | Phage | φLCX | PAPT1 | Cocktail d |
|---|---|---|---|---|
| Cocktail d | Relative AUC (%) | 70.9 ± 7.8 | 51.6 ± 3.9 | 15.7 ± 1.1 |
| | v.s. Cocktail d | * | * | |

| | Phage | φ30-1 | PAi23-JG024 tail ::Plac-PslGh (AB) | Cocktail e |
|---|---|---|---|---|
| Cocktail e | Relative AUC (%) | 30.9 ± 0.8 | 43.3 ± 1.0 | 6.9 ± 0.3 |
| | v.s. Cocktail e | * | * | |

**[Table 3]**

| | Phage | φBrkr | φLCX | PAi23-JG024 tail ::Plac-PslGh (AB) | PAPT1 | Cocktail A |
|---|---|---|---|---|---|---|
| Cocktail A | Relative AUC (%) | 55.1 ± 4.7 | 70.9 ± 7.8 | 43.3 ± 1.0 | 51.6 ± 3.9 | 1.9 ± 0.4 |
| | v.s. Cocktail A | * | * | * | * | |

| | Phage | φBrkr | φLCX2 | PAi23-JG024 tail ::Plac-PslGh (AB)_v3 | PAPT1 | Cocktail B |
|---|---|---|---|---|---|---|
| Cocktail B | Relative AUC (%) | 55.1 ± 4.7 | 63.5 ± 0.8 | 49.8 ± 3.1 | 51.6 ± 3.9 | 2.8 ± 0.5 |
| | v.s. Cocktail B | * | * | * | * | |

| | Phage | φBrkr_FTR2 | φLCX2 | PAi23-JG024 tail ::Plac-PslGh (AB)_v3 | PAPT1 | Cocktail C |
|---|---|---|---|---|---|---|
| Cocktail C | Relative AUC (%) | 78.3 ± 1.4 | 63.5 ± 0.8 | 49.8 ± 3.1 | 51.6 ± 3.9 | 2.7 ± 0.5 |
| | v.s. Cocktail C | * | * | * | * | |

| | Phage | φBrkr | φLCX2 | PAi23-JG024 tail ::Plac-PslGh (AB)_v3 | PAi140 | Cocktail D |
|---|---|---|---|---|---|---|
| Cocktail D | Relative AUC (%) | 55.1 ± 4.7 | 63.5 ± 0.8 | 49.8 ± 3.1 | 99.9 ± 0.6 | 5.3 ± 0.6 |
| | v.s. Cocktail D | * | * | * | * | |

| | Phage | φBrkr | PAi242 | PAi23-JG024 tail ::Plac-PslGh (AB)_v3 | PAPT1 | Cocktail E |
|---|---|---|---|---|---|---|
| Cocktail E | Relative AUC (%) | 55.1 ± 4.7 | 96.0 ± 0.8 | 49.8 ± 3.1 | 51.6 ± 3.9 | 11.9 ± 0.9 |
| | v.s. Cocktail E | * | * | * | * | |

| | Phage | φ30-1 | φLCX2 | PAi23-JG024 tail ::Plac-PslGh (AB)_v3 | PAPT1 | Cocktail F |
|---|---|---|---|---|---|---|
| Cocktail F | Relative AUC (%) | 30.9 ± 0.8 | 63.5 ± 0.8 | 49.8 ± 3.1 | 51.6 ± 3.9 | 2.8 ± 0.6 |
| | v.s. Cocktail F | * | * | * | * | |

| | Phage | φ30-1 | PAi242 | PAi23-JG024 tail ::Plac-PslGh (AB)_v3 | PAi140 | Cocktail G |
|---|---|---|---|---|---|---|
| Cocktail G | Relative AUC (%) | 30.9 ± 0.8 | 96.0 ± 0.8 | 49.8 ± 3.1 | 99.9 ± 0.6 | 9.5 ± 0.9 |
| | v.s. Cocktail G | * | * | * | * | |

| | Phage | φLCX2 | PAi23-JG024 tail ::Plac-PslGh (AB)_v3 | PAPT1 | PAi140 | Cocktail H |
|---|---|---|---|---|---|---|
| Cocktail H | Relative AUC (%) | 63.5 ± 0.8 | 49.8 ± 3.1 | 51.6 ± 3.9 | 99.9 ± 0.6 | 6.7 ± 1.2 |
| | v.s. Cocktail H | * | * | * | * | |

### < Example 12: Biofilm-degrading activity of bacteriophage cocktail >

Whether or not a bacteriophage cocktail has activity of reducing the amount of biofilms formed by *Pseudomonas aeruginosa* was examined. In the same manner as in Example 5, biofilms were formed on pins using the *Pseudomonas aeruginosa* ATCC15692 strain. The bacteriophage cocktail was added to LB liquid medium to the final concentration of 1 × 10⁸ PFU/mL (adjusted to contain each type of bacteriophages at an equal ratio) and brought into contact with the biofilms on the 96-well plate. The experiment was performed in triplicate per condition and repeated four times (4 trials). The average of the 4 trials was determined and one-way analysis of variance and the Dunnett's multiple comparison test were performed. The results demonstrate that Cocktail A (a cocktail of φBrkr, φLCX, PAi23-JG024 tail::Plac-PslGh (AB), and PAPT1) and Cocktail B (a cocktail of φBrkr, φLCX2, PAi23-JG024 tail::Plac-PslGh (AB)_v3, and PAPT1) reduced the biofilm amount to a significant extent (Fig. 12, *p < 0.05).

### < Example 13: Lytic activity of bacteriophage cocktail in infected mouse model >

Mouse-infecting experiments were performed using an ultracentrifugally purified-grade bacteriophage solution by density gradient centrifugation.

### Purification by ultracentrifugation:

DNase I and RNase A were added to the phage lysates to a final concentration of 1 µg/mL, and the resulting mixture was allowed to stand at room temperature for 30 minutes. Bacteriophages were precipitated using Polyethylene Glycol 8000, and the precipitate was collected by centrifugation. Cesium chloride was added to the collected bacteriophage solution at 0.4 g per mL of the solution and gently dissolved therein. The resulting solution was then transferred to an ultracentrifuge vessel. Cesium chloride solutions with densities of 1.3, 1.4, 1.5, and 1.7 g/mL, respectively, were each injected sequentially into the bottom of the vessel using a syringe. The vessel was subjected to centrifugation at 59,000 × g and 4°C for 3 hours, and the bacteriophage-containing band was collected. The collected bacteriophage -containing solution was replaced with phage buffer by dialysis, thereby preparing an ultracentrifugally purified-grade bacteriophage solution.

The respiratory clinical isolate 27-12-PA strain was inoculated into LB liquid medium and cultured with shaking at 37°C for 2.5 hours. The culture solution was centrifuged, the supernatant was discarded, and the precipitate was suspended in physiological saline. The suspension was diluted with physiological saline to adjust the absorbance at 600 nm (OD600) to 0.25 to prepare an inoculum. C57BL/6Jms Slc mice (female, 5-week-old, Japan SLC, Inc.) were infected via oropharyngeal aspiration of 50 µL of the inoculum per mouse. Phage buffer or a bacteriophage cocktail was administered to a mouse by oropharyngeal aspiration at 1 × 10⁹ PFU 1 hour after infection. As the bacteriophage cocktail, Cocktail A (a cocktail of φBrkr, φLCX, PAi23-JG024 tail::Plac-PslGh (AB), and PAPT1), Cocktail B (a cocktail of φBrkr, φLCX2, PAi23-JG024 tail::Plac-PslGh (AB)_v3, and PAPT1), or Cocktail G (a cocktail of φ30-1, PAi242, PAi23-JG024 tail::Plac-PslGh (AB)_v3, and PAi140) was administered. The lungs were extracted approximately 22.5 hours after infection. The lungs were homogenized in 2 ml of physiological saline and washed two times with physiological saline to prepare a tissue homogenate. The tissue homogenate was diluted with physiological saline to prepare 10-fold serially diluted solution. The resultant was applied to LB agar plate and the viable bacterial count was determined. When the viable bacterial count was lower than the detection threshold, the detection threshold was assigned. After the viable bacterial count was converted to a common logarithm, the arithmetic mean was determined, and one-way analysis of variance and the Dunnett's multiple comparison test were performed. Fig. 13 shows the viable bacterial counts in the lungs (log CFU/lung) (N = 10, mean ± standard error). The results demonstrate that the viable bacterial counts in the lungs were reduced to a significant extent in the Cocktail A group, the Cocktail B group, and the Cocktail G group, compared to the phage buffer group (*p < 0.05).

### < Example 14: Culture of bacteriophage using Pseudomonas aeruginosa prophage-lacking strain >

A method of removing lysogenic filamentous phages (e.g., Pf4 and Pf6) from *Pseudomonas aeruginosa,* such as the PAO1 strain, has been reported (Journal of Bacteriology, 2024; 206 (5): e0040223). In short, such method comprises removing filamentous phages by deleting the pflM gene associated with maintenance of the filamentous phages. In accordance with such method, Pf4 and Pf6 were removed from the genome of the *Pseudomonas aeruginosa* ATCC15692 strain (an allied strain of the PAO1 strain) in the manner described below. At the outset, the pUCP18-RedS vector was used to substitute the pflM gene in Pf4 and in Pf6 with the kanamycin resistant gene. The strain lacking the pflM gene was first obtained and a strain lacking both Pf4 and Pf6 was then obtained. Further, a strain from which the pUCP18-RedS vector had been removed was obtained via sucrose treatment. The thus-obtained prophage-lacking strain is referred to as "ATCC15692ΔPf4/Pf6."

The lytic activity of PAi242 against the *Pseudomonas aeruginosa* ATCC15692 strain and ATCC15692ΔPf4/Pf6 strain was examined in the same manner as in Example 1. The culture solution of the *Pseudomonas aeruginosa* ATCC15692 strain or ATCC15692ΔPf4/Pf6 strain cultured overnight in LB liquid medium was mixed with LB liquid medium containing 0.3% soft agar and the mixture was overlaid on LB agar plate. A bacteriophage lysate of PAi242 was diluted to prepare a 1 × 10¹⁰ PFU/mL solution, and the 10-fold serially diluted solutions of the bacteriophage lysates were prepared using the phage buffer. The diluted solutions (2.5 µL each) were added dropwise to the plate and subjected to standing culture at 37°C (the dilution factor of 1 indicates the 1 × 10 ¹⁰ PFU/mL solution in Fig. 14 A). Fig. 14A shows images of lytic plaques appeared after the 10-fold serially diluted solutions of the PAi242 phage were added dropwise to the plate. While PAi242 had lytic activity against the *Pseudomonas aeruginosa* ATCC15692 strain, it did not form any visible plaques, but it formed recognizable plaques against the ATCC15692ΔPf4/Pf6 strain. This indicates that PAi242 sensitivity would be enhanced by removing Pf4 and Pf6 from the genome of the *Pseudomonas aeruginosa* ATCC15692 strain.

Subsequently, the *Pseudomonas aeruginosa* ATCC15692 strain and ATCC15692ΔPf4/Pf6 strain were used to perform bacteriophage liquid culture. These strains were inoculated into LB liquid medium and infected with φBrkr, φ30-1, PAPT1, and PAi242 2.5 hours later with the addition thereof to the final concentration of 1 × 10⁶ PFU/mL. The bacteriophages were cultured with shaking at 37°C for approximately 6 hours to obtain bacteriophage lysates. Liquid culture of bacteriophages was performed in three test tubes, and the titer values of the lysates were determined by the method of Example 10. Fig. 14 B shows the results. In comparison with the use of the *Pseudomonas aeruginosa* ATCC15692 strain, a lysate of PAPT1 exhibiting a 10 times or greater titer value and a lysate of PAi242 exhibiting a 10000 times or greater titer value were obtained when bacteriophage culture was performed using the ATCC15692ΔPf4/Pf6 strain (the prophage-lacking strain) (unpaired t-test, *p < 0.05). The results demonstrate that it is possible to amplify PAPT1 and PAi242 with high efficiency through culture thereof using the ATCC15692ΔPf4/Pf6 strain as the host bacterium.

All publications, patents, and patent applications cited herein are incorporated by reference in their entirety.

### [Accession Nos. (Receipt Nos.)]

Accession No. NITE BP-04040 (the bacteriophage φLCX strain, deposited on December 7, 2023)
Accession No. NITE BP-04031 (the bacteriophage PAPT1 strain, deposited on December 7, 2023)
Accession No. NITE BP-04033 (the bacteriophage PAi140 strain, deposited on December 7, 2023)
Accession No. NITE BP-04036 (the bacteriophage PAi242 strain, deposited on December 7, 2023)
Accession No. NITE BP-04034 (the bacteriophage PAi228 strain, deposited on December 7, 2023)
Accession No. NITE BP-04035 (the bacteriophage PAi239 strain, deposited on December 7, 2023)
Accession No. NITE BP-04032 (the bacteriophage PAi23 strain, deposited on December 7, 2023)
Accession No. NITE BP-04037 (the bacteriophage φBrkr strain, deposited on December 7, 2023)
Accession No. NITE BP-04039 (the bacteriophage φ30-1 strain, deposited on December 7, 2023)
Accession No. NITE BP-04038 (the bacteriophage φBrmt strain, deposited on December 7, 2023)
Receipt No. NITE ABP-04200 (the bacteriophage φLCX2 strain, received on November 13, 2024)
Receipt No. NITE ABP-04201 (the bacteriophage φBrkr_FTR2 strain, received on November 13, 2024)

## Claims

1. A bacteriophage selected from (1) to (8) below:
(1) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04040;
(2) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04031;
(3) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04033;
(4) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04036;
(5) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04034;
(6) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04035;
(7) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04200; and
(8) a bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Receipt No. NITE ABP-04201.

2. The bacteriophage according to claim 1 selected from (1) to (8) below:
(1) a bacteriophage having lytic activity against *Pseudomonas aeruginosa,* wherein a genome of the bacteriophage comprises the nucleic acid sequence of the genome of the bacteriophage identified by Accession No. NITE BP-04040;
(2) a bacteriophage having lytic activity against *Pseudomonas aeruginosa,* wherein a genome of the bacteriophage comprises the nucleic acid sequence of the genome of the bacteriophage identified by Accession No. NITE BP-04031;
(3) a bacteriophage having lytic activity against *Pseudomonas aeruginosa,* wherein a genome of the bacteriophage comprises the nucleic acid sequence of the genome of the bacteriophage identified by Accession No. NITE BP-04033;
(4) a bacteriophage having lytic activity against *Pseudomonas aeruginosa,* wherein a genome of the bacteriophage comprises the nucleic acid sequence of the genome of the bacteriophage identified by Accession No. NITE BP-04036;
(5) a bacteriophage having lytic activity against *Pseudomonas aeruginosa,* wherein a genome of the bacteriophage comprises the nucleic acid sequence of the genome of the bacteriophage identified by Accession No. NITE BP-04034;
(6) a bacteriophage having lytic activity against *Pseudomonas aeruginosa,* wherein a genome of the bacteriophage comprises the nucleic acid sequence of the genome of the bacteriophage identified by Accession No. NITE BP-04035;
(7) a bacteriophage having lytic activity against *Pseudomonas aeruginosa,* wherein a genome of the bacteriophage comprises the nucleic acid sequence of the genome of the bacteriophage identified by Receipt No. NITE ABP-04200; and
(8) a bacteriophage having lytic activity against *Pseudomonas aeruginosa,* wherein a genome of the bacteriophage comprises the nucleic acid sequence of the genome of the bacteriophage identified by Receipt No. NITE ABP-04201.

3. The bacteriophage according to claim 1 or claim 2 selected from (1) to (8) below:
(1) a bacteriophage, wherein a genome of the bacteriophage consists of the nucleic acid sequence of the genome of the bacteriophage identified by Accession No. NITE BP-04040;
(2) a bacteriophage, wherein a genome of the bacteriophage consists of the nucleic acid sequence of the genome of the bacteriophage identified by Accession No. NITE BP-04031;
(3) a bacteriophage, wherein a genome of the bacteriophage consists of the nucleic acid sequence of the genome of the bacteriophage identified by Accession No. NITE BP-04033;
(4) a bacteriophage, wherein a genome of the bacteriophage consists of the nucleic acid sequence of the genome of the bacteriophage identified by Accession No. NITE BP-04036;
(5) a bacteriophage, wherein a genome of the bacteriophage consists of the nucleic acid sequence of the genome of the bacteriophage identified by Accession No. NITE BP-04034;
(6) a bacteriophage, wherein a genome of the bacteriophage consists of the nucleic acid sequence of the genome of the bacteriophage identified by Accession No. NITE BP-04035;
(7) a bacteriophage, wherein a genome of the bacteriophage consists of the nucleic acid sequence of the genome of the bacteriophage identified by Receipt No. NITE ABP-04200; and
(8) a bacteriophage, wherein a genome of the bacteriophage consists of the nucleic acid sequence of the genome of the bacteriophage identified by Receipt No. NITE ABP-04201.

4. A bacteriophage selected from (1) to (8) below:
(1) the bacteriophage identified by Accession No. NITE BP-04040 or a passage strain thereof;
(2) the bacteriophage identified by Accession No. NITE BP-04031 or a passage strain thereof;
(3) the bacteriophage identified by Accession No. NITE BP-04033 or a passage strain thereof;
(4) the bacteriophage identified by Accession No. NITE BP-04036 or a passage strain thereof;
(5) the bacteriophage identified by Accession No. NITE BP-04034 or a passage strain thereof;
(6) the bacteriophage identified by Accession No. NITE BP-04035 or a passage strain thereof;
(7) the bacteriophage identified by Receipt No. NITE ABP-04200 or a passage strain thereof; and
(8) the bacteriophage identified by Receipt No. NITE ABP-04201 or a passage strain thereof.

5. A bacteriophage having lytic activity against *Pseudomonas aeruginosa,* wherein a genome of the bacteriophage (a) comprises a nucleic acid sequence having 90% or more identity to the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa.*

6. The bacteriophage having lytic activity against *Pseudomonas aeruginosa* according to claim 5, wherein a genome of the bacteriophage (a) comprises the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa.*

7. The bacteriophage having lytic activity against *Pseudomonas aeruginosa* according to claim 5, wherein a genome of the bacteriophage (a) consists of the nucleic acid sequence of the genome of the bacteriophage identified by Accession No. NITE BP-04032 except for the region of the tail fiber gene and (b) comprises a nucleic acid sequence of a tail fiber gene comprising at least a part of a tail fiber gene derived from another bacteriophage having lytic activity against *Pseudomonas aeruginosa.*

8. The bacteriophage according to claim 5, wherein a tail fiber gene derived from another bacteriophage comprises the nucleic acid sequence shown in SEQ ID NO: 6 or a nucleic acid sequence having 90% or more identity to SEQ ID NO: 6.

9. A bacteriophage having lytic activity against *Pseudomonas aeruginosa,* wherein a genome of the bacteriophage comprises a nucleic acid sequence(s) encoding at least one protein or active domain selected from (a) to (c) below:
(a) Pyocin G;
(b) PslGh; and
(c) PelAh.

10. The bacteriophage according to claim 9, wherein a genome of the bacteriophage comprises a nucleic acid sequence(s) encoding at least two proteins or active domains selected from (a) to (c) below:
(a) Pyocin G;
(b) PslGh; and
(c) PelAh.

11. The bacteriophage according to claim 9, wherein a genome of the bacteriophage comprises a nucleic acid sequence(s) encoding proteins or active domains (a) to (c) below:
(a) Pyocin G;
(b) PslGh; and
(c) PelAh.

12. A pharmaceutical composition comprising the bacteriophage according to any one of claim 1 to claim 11 and a pharmaceutically acceptable excipient.

13. A pharmaceutical composition comprising at least two types of the bacteriophages according to any one of claim 1 to claim 11 and a pharmaceutically acceptable excipient.

14. A pharmaceutical composition comprising three types of the bacteriophages according to any one of claim 1 to claim 11 and a pharmaceutically acceptable excipient.

15. A pharmaceutical composition comprising four types of the bacteriophages according to any one of claim 1 to claim 11 and a pharmaceutically acceptable excipient.

16. A pharmaceutical composition comprising five types of the bacteriophages according to any one of claim 1 to claim 11 and a pharmaceutically acceptable excipient.

17. The pharmaceutical composition according to any one of claim 12 to claim 16, which is a pharmaceutical composition for preventing or treating infection with *Pseudomonas aeruginosa.*

18. The pharmaceutical composition according to claim 17, wherein the infection with *Pseudomonas aeruginosa* is respiratory infection.

19. A bacteriophage having lytic activity against *Pseudomonas aeruginosa,* wherein a genome of the bacteriophage comprises a nucleic acid sequence having 90% or more identity to the nucleic acid sequence of the genome of the bacteriophage identified by Accession No. NITE BP-04032 except for the region of the tail fiber gene and comprises nucleic acid sequences (A) and (B) below:
(A) a nucleic acid sequence encoding the tail fiber gene derived from bacteriophage JG024; and
(B) a nucleic acid sequence encoding PslGh.

20. A bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6, or a nucleic acid sequence having 90% or more identity to SEQ ID NO: 6; and
(B) the nucleic acid sequence shown in SEQ ID NO: 4, or a nucleic acid sequence having 90% or more identity to SEQ ID NO: 4 and encoding a protein having Psl-degrading activity.

21. A bacteriophage having lytic activity against *Pseudomonas aeruginosa,* wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6; and
(B) the nucleic acid sequence shown in SEQ ID NO: 10 or SEQ ID NO: 11.

22. A pharmaceutical composition comprising a bacteriophage and a pharmaceutically acceptable excipient, wherein the bacteriophage comprises the bacteriophages (i) to (iv) below:
(i) the bacteriophage identified by Accession No. NITE BP-04037 or Receipt No. NITE ABP-04201 or a passage strain thereof;
(ii) the bacteriophage identified by Accession No. NITE BP-04031 or a passage strain thereof;
(iii) a bacteriophage having lytic activity against *Pseudomonas aeruginosa* or a passage strain thereof, wherein a genome of the bacteriophage comprises the nucleic acid sequence of the genome of the bacteriophage identified by Accession No. NITE BP-04032 except for the region of the tail fiber gene and comprises nucleic acid sequences (A) and (B) below:
(A) the nucleic acid sequence shown in SEQ ID NO: 6; and
(B) the nucleic acid sequence shown in SEQ ID NO: 10 or SEQ ID NO: 11; and
(iv) the bacteriophage identified by Accession No. NITE BP-04040 or Receipt No. NITE ABP-04200 or a passage strain thereof.

23. The pharmaceutical composition according to claim 22, which is a pharmaceutical composition for preventing or treating infection with *Pseudomonas aeruginosa.*

24. The pharmaceutical composition according to claim 23, wherein the infection with *Pseudomonas aeruginosa* is respiratory infection.

25. A method for producing a bacteriophage having lytic activity against *Pseudomonas aeruginosa,* the method comprising a step of culturing the bacteriophage in a host bacterium from which a prophage-derived gene has been deleted.
